(19) Europäisches Patentamt
European Patent Office
Office européen des brevets

(11) EP 1 205 480 B1

(12) **EUROPÄISCHE PATENTSCHRIFT**

(45) Veröffentlichungstag und Bekanntmachung des Hinweises auf die Patenterteilung:
**24.11.2010 Patentblatt 2010/47**

(51) Int Cl.:
***C07F 5/02*** *(2006.01)*

(21) Anmeldenummer: **01124917.4**

(22) Anmeldetag: **19.10.2001**

(54) **Tetrakisfluoroalkylborat-Salze und deren Verwendung als Leitsalze**

Tetrakis fluoroalkylborate salts and their use as electrolyte salts

Sels de tétrakisfluoroalkylborate et leur utilisation comme sels électrolytes

(84) Benannte Vertragsstaaten:
**DE FR GB**

(30) Priorität: **10.11.2000 DE 10055811**

(43) Veröffentlichungstag der Anmeldung:
**15.05.2002 Patentblatt 2002/20**

(73) Patentinhaber: **Merck Patent GmbH**
**64271 Darmstadt (DE)**

(72) Erfinder:
- **Schmidt, Michael, Dr.**
**64342 Seeheim-Jugenheim (DE)**
- **Kühner, Andreas, Dr.**
**64289 Darmstadt (DE)**
- **Willner, Helge, Prof. Dr.**
**30926 Seelze (DE)**
- **Bernhardt, Eduard, Dr.**
**47051 Duisburg (DE)**

(74) Vertreter: **Wolff, Felix et al**
**Kutzenberger & Wolff**
**Anwaltssozietät**
**Theodor-Heuss-Ring 23**
**50668 Köln (DE)**

(56) Entgegenhaltungen:
**EP-A- 1 127 888 EP-A- 1 174 941**
**EP-A- 1 229 038**



Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft Tetrakisfluoroalkylborat-Salze, Verfahren zu ihrer Herstellung sowie ihre Verwendung in Elektrolyten, Batterien, Kondensatoren, Superkondensatoren und galvanischen Zellen.

Die Verbreitung von tragbaren elektronischen Geräten, wie z.B. Laptop- und Palmtop-Computern, Mobiltelefonen oder Videokameras und damit auch der Bedarf nach leichten und leistungsfähigen Batterien hat in den letzten Jahren weltweit dramatisch zugenommen.

Angesichts dieses sprunghaft gestiegenen Bedarfs nach Batterien und den damit verbundenen ökologischen Problemen kommt der Entwicklung von wiederaufladbaren Batterien mit einer langen Lebensdauer eine stetig wachsende Bedeutung zu.

Seit den frühen neunziger Jahren werden wiederaufladbare Lithiumionenbatterien kommerziell angeboten. Die meisten dieser Batterien arbeiten mit Lithiumhexafluorophosphat als Leitsalz. Dieses Lithiumsalz stellt jedoch eine extrem hydrolyseempfindliche Verbindung mit einer geringen thermischen Stabilität dar, so daß die entsprechenden Lithiumbatterien aufgrund dieser Eigenschaften des Salzes nur durch sehr aufwendige und somit auch sehr kostenintensive Verfahren hergestellt werden können. Die Empfindlichkeit dieses Lithiumsalzes mindert zudem die Lebensdauer sowie die Leistung dieser Lithiumbatterien und beeinträchtigt auch deren Einsatz unter extremen Bedingungen, wie z.B. hohen Temperaturen.

Es hat daher an Versuchen nicht gefehlt, Lithiumsalze mit verbesserten Eigenschaften zur Verfügung zu stellen. So beschreiben die US 4,505,997 und US 5,273,840 die Verwendung von Lithium-[bis(trifluormethylsulfonyl)imid] bzw. Lithium[tris(trifluormethylsulfonyl)methanid]-Salzen als Leitsalze in Batterien. Beide Salze zeigen eine hohe anodische Stabilität und bilden mit organischen Carbonaten Lösungen mit hoher Leitfähigkeit aus. Lithium[bis(trifluormethylsulfonyl) imid] hat jedoch den Nachteil, daß es das Aluminiummetall, das als kathodischer Stromableiter in Lithiumbatterien fungiert, nicht in ausreichendem Maße passiviert. Lithium[tris(trifluormethylsulfonyl)methanid] dagegen läßt sich nur durch einen sehr hohen Aufwand herstellen und reinigen, so daß die Verwendung dieses Salzes als Leitsalz in Batterien die Produktionskosten für solche Lithiumbatterien stark erhöht.

Ferner sind aus EP 117 4 941 (Dokument nach Artikel 54(3) EPÜ) Alkaki-und Ammonium-Salze von Tetrafluoroalkylboraten bekannt.

Ein weiteres Lithiumsalz, das in Batteriezellen zur Anwendung kommt, ist Lithiumtetrafluoroborat. Dieses Salz ist jedoch in den meisten Lösungsmitteln nur relativ schwer löslich, so daß dessen Lösungen im allgemeinen nur eine geringe ionische Leitfähigkeit aufweisen.

Es war daher die Aufgabe der vorliegenden Erfindung, Leitsalze zur Verfügung zu stellen, die über einen langen Zeitraum keine oder nur geringste Anzeichen einer hydrolytischen Zersetzung zeigen. Desweiteren sollen diese Leitsalze auch eine hohe ionische Leitfähigkeit, eine hohe thermische Stabilität und eine gute bis sehr gute Löslichkeit in den gängigen Lösungsmitteln aufweisen. Eine weitere Aufgabe der vorliegenden Erfindung bestand darin, die Lebensdauer und die Leistung von primären und sekundären Batterien, Kondensatoren, Superkondensatoren und/oder galvanischen Zellen zu verlängern bzw. zu verbessern.

Diese Aufgabe wird durch die Bereitstellung von Tetrakisfluoroalkylborat-Salzen der allgemeinen Formel (I) gelöst,

$$M^{n+}\ ([BR_4]^-)_n \qquad (I)$$

worin

$M^{n+}$ ein einwertiges, zweiwertiges oder dreiwertiges Kation ist, die Liganden R jeweils gleich und geradkettig oder verzweigt sind und für ($C_xF2_{x+1}$) mit $1 \leq x \leq 8$ stehen und n = 1, 2 oder 3 ist

wobei Tetrakisfluoroalkylborat-Salze ausgenommen sind, worin $M^{n+}$ für ein Alkalimetall-Kation oder für ein Ammonium-Ion der allgemeinen Formel $NR'_4$ steht, wobei R' jeweils unabhängig voneinander für H, Alkyl-, Alkenyl- oder Aryl-Gruppen steht, wenn die Liganden R jeweils für ($C_xF_{2x+1}$) mit $1 \leq x \leq 4$ stehen.

Bevorzugt sind erfindungsgemäße Tetrakisfluoroalkylborat-Salze der allgemeinen Formel (I), in denen das Kation $M^{n+}$ ein Alkalimetall-Kation, vorzugsweise ein Lithium-, Natrium- oder Kalium-Kation, besonders bevorzugt ein Lithium-Kation, ein Magnesium- oder Aluminium-Kation ist.

Desweiteren sind auch Tetrakisfluoroalkylborat-Salze der allgemeinen Formel (I) bevorzugt, in denen das Kation $M^{n+}$ ein organisches Kation, vorzugsweise ein Nitrosyl-Kation, ein Nitryl-Kation oder ein Kation der allgemeinen Formel $[N(R^7)_4]^+$, $[P(N(R^7)_2)_kR_{4-k}]^+$ mit $0 \leq k \leq 4$ oder $[C(N(R^7)_2)^3]^+$ ist, wobei die Reste $R^7$, jeweils gleich oder verschieden sind, und für

H,

$$C_oF_{2o+1-p-q}H_pA_q$$

oder worin

$1 \leq o \leq 10$, $0 \leq p \leq 2o+1$ und $0 \leq q \leq 2o+1$, vorzugsweise $1 \leq o \leq 6$, $0 \leq p \leq 2o+1$ und $0 \leq q \leq 2o+1$, sind und A jeweils einen gegebenenfalls Heteroatome aufweisenden aromatischen Rest oder einen vorzugsweise 5- oder 6-gliedrigen Cycloalkyl-Rest bedeutet.

Als aromatischer oder cycloaliphatischer Rest A, der gegebenenfalls Heteroatome aufweisen kann, können alle dem Fachmann bekannten, zur Herstellung von $[N(R^7)_4]^+$, $[P(N(R^7)_2)_kR_{4-k}]^+$ mit $0 \leq k \leq 4$ oder $[C(N(R^7)_2)_3]^+$-Kationen geeigneten Aromaten, Heteroaromaten oder Cycloaliphaten eingesetzt werden.

Vorzugsweise steht A für einen 5- oder 6-gliedrigen, gegebenenfalls Stickstoff- und/oder Schwefel- und/oder Sauerstoffatome aufweisenden aromatischen Rest, besonders bevorzugt für einen Phenyl- oder Pyridin-Rest.

In einer weiteren bevorzugten Ausführungsform der vorliegenden Erfindung ist das Kation $M^{n+}$ ein heteroaromatisches Kation ausgewählt aus der Gruppe der heteroaromatischen Kationen der allgemeinen Formel (II) bis (IX):

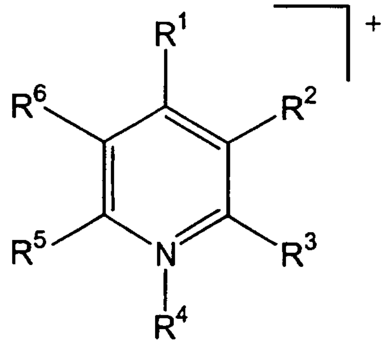

(II)

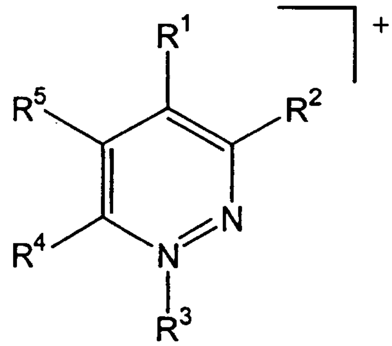

(III)

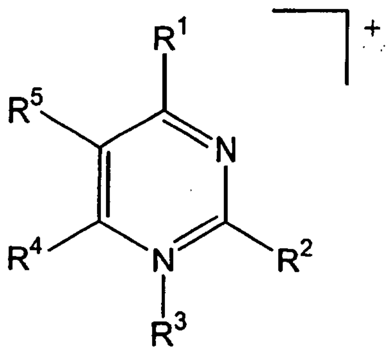

(IV)

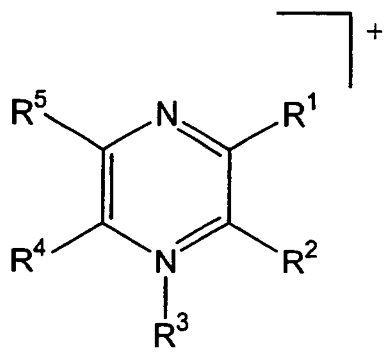

(V)

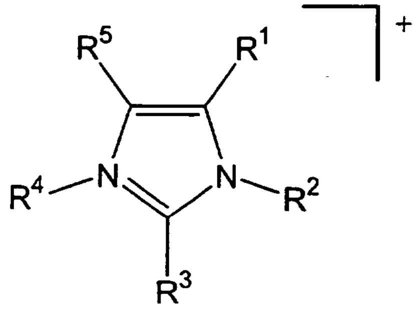

(VI)

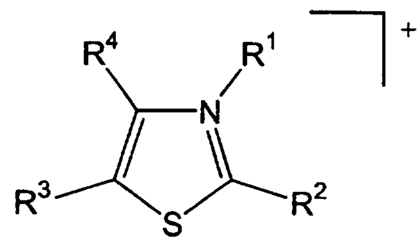

(VII)

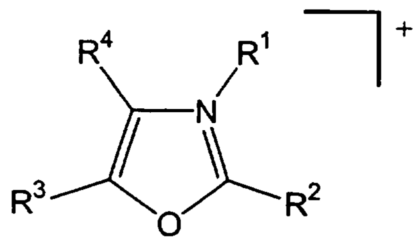

(VIII)

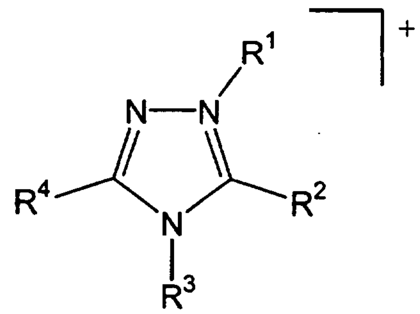

(IX)

Die Reste $R^1$ bis $R^6$, die jeweils gleich oder verschieden sein können, stehen für einen H-Rest, einen Halogen-Rest, vorzugsweise einen Fluor-Rest, oder für einen $C_{1-8}$-Alkyl-Rest, der gegebenenfalls mit den Substituenten F, Cl, $N(C_aF_{(2a+1-b)}H_b)_2$, $O(C_aF_{(2a+1-b)}H_b)$, $SO_2(C_aF_{(2a+1-b)}H_b)$ oder $C_aF_{(2a+1-b)}H_b$ substituiert sein kann, in denen $1 \leq a \leq 6$ und $0 \leq b \leq 2a+1$ bedeuten.

Es können auch zwei der Reste $R^1$ bis $R^6$ gemeinsam einen $C_{1-8}$-Alkyl-Rest bedeuten, der gegebenenfalls mit den Substituenten F, Cl, $N(C_aF_{(2a+1-b)}H_b)_2$, $O(C_aF_{(2a+1-b)}H_b)$, $SO_2(C_aF_{(2a+1-b)}H_b)$ oder $C_aF_{(2a+1-b)}H_b$ substituiert sein kann, in denen $1 \leq a \leq 6$ und $0 \leq b \leq 2a+1$ bedeuten.

Ebenfalls bevorzugt sind Tetrakisfluoroalkylborat-Salze der allgemeinen Formel (I), in denen die Liganden R jeweils gleich sind und für $(C_xF_{2x+1})$ stehen und x = 1 oder 2 bedeutet. Besonders bevorzugt sind solche Tetrakisfluoroalkylborat-Salze, in denen die Liganden R jeweils gleich sind und für einen $CF_3$-Rest stehen.

Die erfindungsgemäßen Salze der allgemeinen Formel (I) können sowohl in reiner Form als auch in Form ihrer Mischungen als Leitsalze in Elektrolyten, primären und sekundären Batterien, Kondensatoren, Superkondensatoren und/oder galvanischen Zellen eingesetzt werden. Es ist ebenfalls möglich, die erfindungsgemäßen Salze in Mischung mit weiteren, dem Fachmann bekannten Lithiumsalzen als Leitsalze zu verwenden.

Sie können in Anteilen zwischen 1 und 99% in Kombination mit anderen Leitsalzen, die in elektrochemischen Zellen Anwendung finden, verwendet werden. Geeignet sind z.B. Leitsalze ausgewählt aus der Gruppe $LiPF_6$, $LiBF_4$, $LiClO_4$, $LiAsF_6$, $LiCF_3SO_3$, $LiN(CF_3SO_2)_2$ oder $LiC(CF_3SO_2)_3$ und Mischungen aus wenigstens zwei dieser Verbindungen.

Ebenso können die Salze der Formel (I) und deren Mischungen in Elektrolyten für elektrochemische Zellen verwendet werden.

Die Elektrolyte können auch organische Isocyanate (DE 100 42 149) zur Herabsetzung des Wassergehaltes enthalten. Auch Verbindungen der allgemeinen Formel

$$[([R^1(CR^2R^3)_k]_lA_x)_yKt]^+ \; {}^-N(CF_3)_2$$

wobei

Kt N, P, As, Sb, S, Se
A N, P, P(O), O, S, S(O), $SO_2$, As, As(O), Sb, Sb(O)

$R^1$, $R^2$ und $R^3$
gleich oder verschieden
H, Halogen, substituiertes und/oder unsubstituiertes Alkyl $C_nH_{2n+1}$, substituiertes und/oder unsubstituiertes Alkenyl mit 1-18 Kohlenstoffatomen und einer oder mehreren Doppelbindungen, substituiertes und/oder unsubstituiertes Alkinyl mit 1-18 Kohlenstoffatomen und einer oder mehreren Dreifachbindungen, substituiertes und/oder unsubstituiertes Cycloalkyl $C_mH_{2m-1}$ , ein- oder mehrfach substituiertes und/oder unsubstituiertes Phenyl, substituiertes und/oder unsubstituiertes Heteroaryl,
A kann in verschiedenen Stellungen in $R^1$, $R^2$ und/oder $R^3$ eingeschlossen sein,
Kt kann in cyclischen oder heterocyclischen Ring eingeschlossen sein, die an Kt gebundenen Gruppen können gleich oder verschieden sein mit

n 1-18
m 3-7
k 0, 1-6
l 1 oder 2 im Fall von x=1 und 1 im Fall x=0
x 0,1
y 1-4

bedeuten, können enthalten sein (DE 9941566). Das Verfahren zur Herstellung der Verbindungen ist dadurch gekennzeichnet, daß ein Alkalisalz der allgemeinen Formel

$$D^{+}\ {}^{-}N(CF_3)_2$$

mit $D^+$ ausgewählt aus der Gruppe der Alkalimetalle in einem polaren organischen Lösungsmittel mit einem Salz der allgemeinen Formel

$$[([R^1(CR^2R^3)_k]_lA_x)_yKt]^{+}\ {}^{-}E$$

wobei
Kt, A, $R^1$, $R^2$, $R^3$, k, l, x und y die oben angegebene Bedeutung haben und $^-E$ $F^-$, $Cl^-$, $Br^-$, $I^-$, $BF_4^-$, $ClO_4^-$, $AsF_6^-$, $SbF_6^-$ oder $PF_6^-$
bedeutet, umgesetzt wird.
Die erfindungsgemäßen Verbindungen können auch in Elektrolyten enthalten sein, die Verbindungen der Formel

$$X\text{-}(CYZ)_m\text{-}SO_2N(CR^1R^2R^3)_2$$

mit

X H, F, Cl, $C_nF_{2n+1}$, $C_nF_{2n-1}$, $(SO_2)_kN(CR^1R^2R^3)_2$
Y H, F, Cl
Z H, F, Cl
$R^1$, $R^2$, $R^3$ H und/oder Alkyl, Fluoralkyl, Cycloalkyl
m 0-9 und falls X=H, m≠0
n 1-9
k 0, falls m=0 und k=1, falls m=1-9

enthalten, dagestellt dadurch, daß teil- oder perfluorierte Alkysulfonylfluoride mit Dimethylamin in organischen Lösungsmitteln umgesetzt werden (DE 19953051).
Auch Lithiumkomplexsalze der Formel

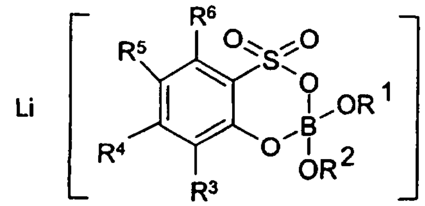

wobei

$R^1$ und $R^2$ gleich oder verschieden sind, gegebenenfalls durch eine Einfach- oder Doppelbildung direkt miteinander verbunden sind, jeweils einzeln oder gemeinsam die Bedeutung eines aromatischen Rings aus der Gruppe Phenyl, Naphthyl, Anthracenyl oder Phenanthrenyl, der unsubstituiert oder ein- bis sechsfach durch Alkyl ($C_1$ bis $C_6$), Alkoxygruppen ($C_1$ bis $C_6$) oder Halogen (F, Cl, Br) substituiert sein kann, haben,
oder jeweils einzeln oder gemeinsam die Bedeutung eines aromatischen heterocyclischen Rings aus der Gruppe Pyridyl, Pyrazyl oder Pyrimidyl, der unsubstituiert oder ein- bis vierfach durch Alkyl ($C_1$ bis $C_6$), Alkoxygruppen ($C_1$ bis $C_6$) oder Halogen (F, Cl, Br) substituiert sein kann, haben,
oder jeweils einzeln oder gemeinsam die Bedeutung eines aromatischen Rings aus der Gruppe Hydroxylbenzoecarboxyl, Hydroxylnaphthalincarboxyl, Hydroxylbenzoesulfonyl und Hydroxylnaphthalinsulfonyl, der unsubstituiert oder ein- bis vierfach durch Alkyl ($C_1$ bis $C_6$), Alkoxygruppen ($C_1$ bis $C_6$) oder Halogen (F, Cl, Br) substituiert sein kann, haben,
$R^3$-$R^6$ können jeweils einzeln oder paarweise, gegebenenfalls durch eine Einfach- oder Doppelbindung direkt miteinander verbunden, folgende Bedeutung haben:

1. Alkyl ($C_1$ bis $C_6$),Alkyloxy ($C_1$ bis $C_6$) oder Halogen (F, Cl, Br)
2. ein aromatischer Ring aus den Gruppen

Phenyl, Naphthyl, Anthracenyl oder Phenanthrenyl, der unsubstituiert oder einbis sechsfach durch Alkyl ($C_1$ bis $C_6$), Alkoxygruppen ($C_1$ bis $C_6$) oder Halogen (F, Cl, Br) substituiert sein kann,
Pyridyl, Pyrazyl oder Pyrimidyl, der unsubstituiert oder ein- bis vierfach durch Alkyl ($C_1$ bis $C_6$), Alkoxygruppen ($C_1$ bis $C_6$) oder Halogen (F, Cl, Br) substituiert sein kann,
die über folgendes Verfahren (DE 199 32 317) dargestellt werden

a) 3-, 4-, 5-, 6-substituiertes Phenol in einem geeigneten Lösungsmittel mit Chlorsulfonsäure versetzt wird,
b) das Zwischenprodukt aus a) mit Chlortrimethylsilan umgesetzt, filtriert und fraktioniert destilliert wird,
c) das Zwischenprodukt aus b) mit Lithiumtetramethanolat-borat(1-), in einem geeigneten Lösungsmittel umgesetzt und daraus das Endprodukt isoliert wird, können im Elektrolyten enthalten sein.

Auch Elektrolyte mit Komplexsalzen der allgemeinen Formel (DE 199 51 804)

$$M^{x+}[EZ]^{y-}_{x/y}$$

worin bedeuten:

x,y 1,2,3,4,5,6
$M^{x+}$ ein Metallion
E eine Lewis-Säure, ausgewählt aus der Gruppe

$BR^1R^2R^3$, $AlR^1R^2R^3$, $PR^1R^2R^3R^4R^5$, $AsR^1R^2R^3R^4R^5$, $VR^1R^2R^3R^4R^5$,
$R^1$ bis $R^5$ gleich oder verschieden, gegebenenfalls durch eine Einfach- oder Doppelbildung direkt miteinander verbunden sind, jeweils einzeln oder gemeinsam die Bedeutung
eines Halogens (F, Cl, Br),
eines Alkyl- oder Alkoxyrestes ($C_1$ bis $C_8$) der teilweise oder vollständig durch F, Cl, Br substituiert sein kann,
eines, gegebenenfalls über Sauerstoff gebundenen aromatischen Rings aus der Gruppe Phenyl, Naphthyl, Anthracenyl oder Phenanthrenyl, der unsubstituiert oder ein- bis sechsfach durch Alkyl ($C_1$ bis $C_8$) oder F, Cl, Br substituiert sein kann
eines, gegebenenfalls über Sauerstoff gebundenen aromatischen heterocyclischen Rings aus der Gruppe Pyridyl, Pyrazyl oder Pyrimidyl, der unsubstituiert oder ein- bis vierfach durch Alkyl ($C_1$ bis $C_8$) oder F, Cl, Br substituiert sein kann,
haben können und

Z $OR^6$, $NR^6R^7$, $CR^6R^7R^8$, $OSO_2R^6$, $N(SO_2R^6)(SO_2R^7)$, $C(SO_2R^6)(SO_2R^7)(SO_2R^8)$, $OCOR^6$, wobei

$R^6$ bis $R^8$ gleich oder verschieden sind, gegebenenfalls durch eine Einfach- oder Doppelbindung direkt miteinander verbunden sind, jeweils einzeln oder gemeinsam die Bedeutung eines Wasserstoffs oder die Bedeutung wie $R^1$ bis $R^5$ haben, hergestellt durch Umsetzung von einem entsprechenden Bor- oder Phosphor-Lewis-Säure-Solvenz-Adukt mit einem Lithium- oder Tetraalkylammonium-Imid, -Methanid oder -Triflat, können verwendet werden.

Auch Boratsalze (DE 199 59 722) der allgemeinen Formel worin bedeuten:

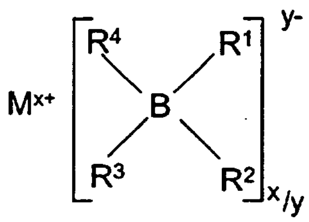

M ein Metallion oder Tetraalkylammoniumion

x,y 1, 2, 3, 4, 5 oder 6

$R^1$ bis $R^4$ gleich oder verschieden, gegebenenfalls durch eine Einfach- oder Doppelbindung direkt miteinander verbundener Alkoxy- oder Carboxyreste ($C_1$-$C_8$) können enthalten sein. Hergestellt werden diese Boratsalze durch Umsetzung von Lithiumtetraalkoholatborat oder einem 1:1 Gemisch aus Lithiumalkoholat mit einem Borsäureester in einem aprotischen Lösungsmittel mit einer geeigneten Hydroxyl- oder Carboxylverbindung im Verhältnis 2:1 oder 4:1.

Auch Additive wie Silanverbindungen der allgemeinen Formel

$SiR^1R^2R^3R^4$

mit $R^1$ bis $R^4$ H

$C_yF_{2y+1-z}H_z$

$OC_yF_{2y+1-z}H_z$

$OC(O)C_yF_{2y+1-z}H_z$

$OSO_2C_yF_{2y+1-z}H_z$

und

$$1 \leq x < 6$$

$$1 \leq y \leq 8$$

und

$$0 \leq z \leq 2y+1$$

und

$R^1$-$R^4$ gleich oder verschieden
mit der Bedeutung eines aromatischen Rings aus der Gruppe Phenyl, Naphthyl der unsubstituiert oder ein- oder mehrfach durch F, $C_yF_{2y+1-z}H_z$ oder $OC_yF_{2y+1-z}H_z$, $OC(O)C_yF_{2y+1-z}H_z$, $OSO_2C_yF_{2y+1-z}H_z$, $N(C_nF_{2n+1-z}H_z)_2$ substituiert sein kann, oder
mit der Bedeutung eines heterocyclischen aromatischen Rings aus der Gruppe Pyridyl, Pyrazyl oder Pyrimidyl, die jeweils ein- oder mehrfach mit F, $C_yF_{2y+1-z}H_z$ oder $OC_yF_{2y+1-z}H_z$, $OC(O)C_yF_{2y+1-z}H_z$, $OSO_2C_yF_{2y+1-z}H_z$, $N(C_nF_{2n+1-z}H_z)_2$ substituiert sein können (DE 100 276 26), können enthalten sein.

Die erfindungsgemäßen Verbindungen können auch in Elektrolyte eingesetzt werden, die Lithiumfluoralkylphosphate der folgenden Formel enthalten,

$$Li^+[PF_x(C_yF_{2y+1-z}H_z)_{6-x}]^-$$

worin

$$1 \leq x \leq 5$$

$$3 \leq y \leq 8$$

$$0 \leq z \leq 2y + 1$$

bedeuten und die Liganden $(C_yF_{2y+1-z}H_z)$ gleich oder verschieden sein können, wobei die Verbindungen der allgemeinen Formel,

$$Li^+[PF_a(CH_bF_c(CF_3)_d)_e]^-$$

in der a eine ganze Zahl von 2 bis 5, b = 0 oder 1, c = 0 oder 1, d = 2 und e eine ganze Zahl von 1 bis 4 bedeuten, mit den Bedingungen, daß b und c nicht gleichzeitig jeweils = 0 bedeuten und die Summe aus a + e gleich 6 ist und die Liganden $(CH_bF_c(CF_3)_d)$ gleich oder verschieden sein können, ausgenommen sind (DE 100 089 55). Das Verfahren zur Herstellung von Lithiumfluoralkylphosphaten ist dadurch gekennzeichnet, daß wenigstens eine Verbindung der allgemeinen Formel

$$H_mP(C_nH_{2n+1})_{3-m} \quad (III),$$

$$OP(C_nH_{2n+1})_3 \quad (IV),$$

$$Cl_mP(C_nH_{2n+1})_{3-m} \quad (V),$$

$$F_mP(C_nH_{2n+1})_{3-m} \quad (VI),$$

$$Cl_oP(C_nH_{2n+1})_{5-o} \quad (VII),$$

$$F_oP(C_nH_{2n+1})_{5-o} \quad (VIII),$$

in denen jeweils
$0 < m < 2$, $3 < n < 8$ und $0 < o < 4$ bedeutet
durch Elektrolyse in Fluorwasserstoff fluoriert wird, das so erhaltene Gemisch der Fluorierungsprodukte durch Extraktion, Phasentrennung und/oder Destillation aufgetrennt wird, und das so erhaltene fluorierte Alkylphosphoran in einem aprotischen Lösungsmittel oder Lösungsmittelgemisch unter Feuchtigkeitsausschluß mit Lithiumfluorid umgesetzt wird, und das so erhaltene Salz nach den üblichen Methoden gereinigt und isoliert wird.
Die erfindungsgemäßen Verbindungen können auch in Elektrolyten eingesetzt werden, die Salze der Formel

$$Li[P(OR^1)_a(OR^2)_b(OR^3)_c(OR^4)_dF_e]$$

worin $0 < a+b+c+d \leq 5$ und $a+b+c+d+e=6$ gilt, und $R^1$ bis $R^4$ unabhängig voneinander Alkyl-, Aryl- oder Heteroarylreste

sind, wobei mindestens zwei von $R^1$ bis $R^4$ durch eine Einfach- oder Doppelbindung direkt miteinander verbunden sein können, enthalten (DE 100 16 801). Dargestellt werden die Verbindungen durch Umsetzung von Phosphor (V)-Verbindungen der allgemeinen Formel

$$P(OR^1)_a(OR^2)_b(OR^3)_c(OR^4)_dF_e$$

worin $0 < a+b+c+d \leq 5$ und $a+b+c+d+e=5$ gilt, und $R^1$ bis $R^4$ die oben angegebenen Bedeutungen haben mit Lithiumfluorid in Gegenwart eines organischen Lösungsmittels.

Auch Ionische Flüssigkeiten der allgemeinen Formel

$$K^+A^-$$

worin bedeuten:

$K^+$ ein Kation ausgewählt aus der Gruppe

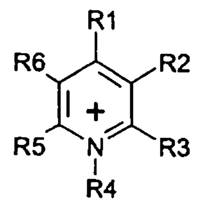
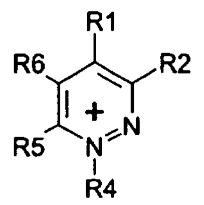

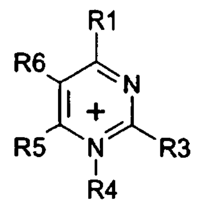
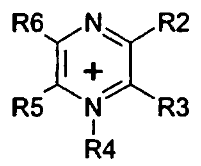

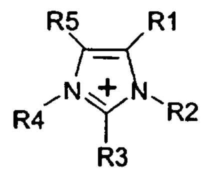
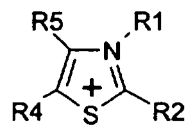

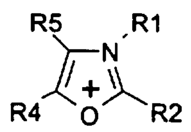
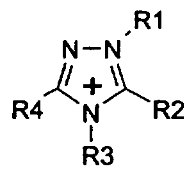

wobei $R^1$ bis $R^5$ gleich oder verschieden, gegebenenfalls durch eine Einfach- oder Doppelbindung direkt miteinander verbunden sind und jeweils einzeln oder gemeinsam folgende Bedeutung haben:

- H,
- Halogen,
- Alkylrest ($C_1$ bis $C_8$) der teilweise oder vollständig durch weitere Gruppen, vorzugsweise F, Cl, $N(C_nF_{(2n+1-x)}H_x)_2$, $O(C_nF_{(2n+1-x)}H_x)$, $SO_2(C_nF_{(2n+1-x)}H_x)$, $C_nF_{(2n+1-x)}H_x$ mit $1<n<6$ und $0<x\leq13$ substituiert sein kann

und

$A^-$ ein Anion ausgewählt aus der Gruppe

$$[B(OR^1)_n(OR^2)_m(OR^3)_o(OR^4)_p]^-$$

mit $0 \leq n, m, o, p \leq 4$ und
$m+n+o+p=4$
wobei $R^1$ bis $R^4$ verschieden oder paarweise gleich sind, gegebenenfalls durch eine Einfach- oder Doppelbildung direkt miteinander verbunden sind, jeweils einzeln oder gemeinsam
die Bedeutung eines aromatischen Rings aus der Gruppe Phenyl, Naphthyl, Anthracenyl oder Phenanthrenyl, der unsubstituiert oder ein- oder mehrfach durch $C_nF_{(2n+1-x)}H_x$ mit $1<n<6$ und $0<x\leq 13$ oder Halogen (F, Cl, Br) substituiert sein kann, besitzen,
die Bedeutung eines aromatischen heterocyclischen Rings aus der Gruppe Pyridyl, Pyrazyl oder Pyrimidyl, der unsubstituiert oder ein- oder mehrfach durch $C_nF_{(2n+1-x)}H_x$ mit $1<n<6$ und $0<x\leq 13$ oder Halogen oder Halogen (F, Cl, Br) substituiert sein kann, besitzen,
die Bedeutung eines Alkylrests ($C_1$ bis $C_8$) der teilweise oder vollständig durch weitere Gruppen, vorzugsweise F, Cl, , $N(C_nF_{(2n+1-x)}H_x)_2$, $O(C_nF_{(2n+1-x)}H_x)$, $SO_2(C_nF_{(2n+1-x)}H_x)$, $C_nF_{(2n+1-x)}H_x$ mit $1<n<6$ und $0<x\leq 13$ substituiert sein kann, besitzen,
beziehungsweise $OR^1$ bis $OR^4$
einzeln oder gemeinsam die Bedeutung eines aromatischen oder aliphatischen Carboxyl-, Dicarboxyl-, Oxysulfonyl- oder Oxycarboxylrests, der teilweise oder vollständig durch weitere Gruppen, vorzugsweise F, Cl, , $N(C_nF_{(2n+1-x)}H_x)_2$, $O(C_nF_{(2n+1-x)}H_x)$, $SO_2(C_nF_{(2n+1-x)}H_x)$, $C_nF_{(2n+1-x)}H_x$ mit $1<n<6$ und $0<x\leq 13$ substituiert sein kann, besitzen (DE 100 265 65), können im Elektrolyten enthalten sein. Auch Ionische Flüssigkeiten $K^+A^-$ mit $K^+$ definiert wie oben und

$A^-$ ein Anion ausgewählt aus der Gruppe

$$\left[ PF_x(C_yF_{2y+1-z}H_z)_{6-x} \right]^-$$

und
$1 \leq x < 6$
$1 \leq y \leq 8$ und
$0 \leq z \leq 2y+1$
können enthalten sein (DE 100 279 95).

Die erfindungsgemäßen Verbindungen können in Elektrolyte für elektrochemische Zellen eingesetzt werden, die Anodenmaterial, bestehend aus beschichteten Metallkernen, ausgewählt aus der Gruppe Sb, Bi, Cd, In, Pb, Ga und Zinn oder deren Legierungen, enthalten (DE 100 16 024). Das Verfahren zur Herstellung dieses Anodenmaterials ist dadurch gekennzeichnet, daß

a) eine Suspension oder ein Sol des Metall- oder Legierungskerns in Urotropin hergestellt wird,
b) die Suspension mit Kohlenwasserstoffen mit $C_5$-$C_{12}$ emulgiert werden,
c) die Emulsion auf die Metall- oder Legierungskerne aufgefällt werden und
d) durch Temperung des Systems die Metallhydroxide bzw. -oxihydroxide in das entsprechende Oxid übergeführt werden.

Die erfindungsgemäßen Verbindungen können auch in Elektrolyte für elektrochemische Zellen eingesetzt werden, mit Kathoden aus gängigen Lithium-Interkalations und Insertionsverbindungen aber auch mit Kathodenmaterialien, die aus Lithium-Mischoxid-Partikel bestehen, die mit einem oder mehreren Metalloxiden (DE 199 22 522) beschichtet sind, indem die Partikel in einem organischen Lösungsmittel suspendiert werden, die Suspension mit einer Lösung einer hydrolisierbaren Metallverbindung und einer Hydrolyselösung versetzt und danach die beschichteten Partikel abfiltriert, getrocknet und gegebenenfalls calciniert werden. Sie können auch aus Lithium-Mischoxid-Partikel bestehen, die mit einem oder mehreren Polymeren (DE 199 46 066) beschichtet sind, erhalten durch ein Verfahren, bei dem die Partikel in einem Lösungsmittel suspendiert werden und anschließend die beschichteten Partikel abfiltriert, getrocknet und gegebenenfalls calciniert werden. Ebenso können die erfindungsgemäßen Verbindungen in Systemen mit Kathoden eingesetzt werden, die aus Lithium-Mischoxid-Partikeln bestehen, die mit Alkalimetallverbindungen und Metalloxiden ein- oder mehrfach beschichtet sind (DE 100 14 884). Das Verfahren zur Herstellung dieser Materialien ist dadurch gekennzeichnet, daß die Partikel in einem organischen Lösungsmittel suspendiert werden, eine Alkalimetallsalzverbindung suspendiert in einem organischen Lösungsmittel zugegeben wird, Metalloxide gelöst in einem organischen Lösungsmittel zugegeben werden, die Suspension mit einer Hydrolyselösung versetzt wird und anschließend die beschichteten Partikel abfiltriert, getrocknet und calciniert werden. Ebenso können die erfindungsgemäßen Verbindungen in Systemen eingesetzt werden, die Anodenmaterialien mit dotiertem Zinnoxid enthalten (DE 100 257 61). Dieses Anodenmaterial wird hergestellt indem

a) eine Zinnchlorid-Lösung mit Harnstoff versetzt wird,
b) die Lösung mit Urotropin und einer geeigneten Dotierverbindung versetzt wird,
c) das so erhaltene Sol in Petrolether emulgiert wird,
d) das erhaltene Gel gewaschen und das Lösungsmittel abgesaugt sowie
e) das Gel getrocknet und getempert wird.

Ebenso können die erfindungsgemäßen Verbindungen in Systemen eingesetzt werden, die Anodenmaterialien mit reduziertem Zinnoxid enthalten (DE 100 257 62). Dieses Anodenmaterial wird hergestellt indem

a) eine Zinnchlorid-Lösung mit Harnstoff versetzt wird,
b) die Lösung mit Urotropin versetzt wird,
c) das so erhaltene Sol in Petrolether emulgiert wird,
d) das erhaltene Gel gewaschen und das Lösungsmittel abgesaugt wird,
e) das Gel getrocknet und getempert wird und
f) das erhaltene $SnO_2$ in einem begasbaren Ofen einem reduzie-rendem Gasstrom ausgesetzt wird.

Vorzugsweise werden die erfindungsgemäßen Salze in reiner Form als Leitsalze verwendet, da auf diese Weise eine besonders gute Reproduzierbarkeit der elektrochemischen Eigenschaften gewährleistet werden kann.

Ein weiterer Gegenstand der Erfindung ist auch ein Verfahren zur Herstellung von erfindungsgemäßen Tetrakisfluoroalkylborat-Salzen der allgemeinen Formel (I), in denen die Liganden R jeweils identisch sind und für einen $CF_3$-Rest stehen.

Bei diesem Verfahren wird wenigstens ein Salz der allgemeinen Formel (X),

$$M^{n+}\ ([B(CN)_4]^-)_n \qquad (X)$$

worin $M^{n+}$ und n die oben angegebene Bedeutung haben, durch Umsetzung mit wenigstens einem Fluorierungsmittel in wenigstens einem Lösungsmittel fluoriert und die so erhaltene fluorierte Verbindung der allgemeinen Formel (I) nach üblichen, dem Fachmann bekannten Methoden gereinigt und isoliert.

Die so erhaltenen Tetrakisfluoroalkylborat-Salze besitzen unmittelbar nach der Fluorierung häufig eine Reinheit von > 99%. Sofern dies erforderlich sein sollte, kann eine weitere Aufreinigung der Salze nach üblichen, dem Fachmann bekannten Methoden, beispielsweise durch Umkristallisation in einem geeigneten Lösungsmittel oder Lösungsmittelgemisch erfolgen. Die Auswahl geeigneter Lösungsmittel bzw. Lösungsmittelgemische ist dem Fachmann mittels einfacher Vorversuche möglich.

Die Synthese der Verbindungen der allgemeinen Formel (X) kann analog zu der in E. Bernhardt, G. Henkel, H. Willner Z. Anorg. Allg. Chem., 2000, Vol. 626, Seite 560 veröffentlichten Methode erfolgen. Diese Literatur wird hiermit als Referenz eingeführt und gilt als Teil der Offenbarung.

Bei dem erfindungsgemäßen Verfahren erfolgt die Umsetzung mit dem Fluorierungsmittel vorzugsweise bei einer Temperatur im Bereich von -80 bis +20°C, besonders bevorzugt bei einer Temperatur im Bereich von -60 °C bis 0 °C.

Als geeignete Fluorierungsmittel werden in dem erfindungsgemäßen Verfahren bevorzugt Fluor, Chlorfluorid, Chlortrifluorid, Chlorpentafluorid, Bromtrifluorid, Brompentafluorid oder ein Gemisch aus wenigstens zwei dieser Fluorierungsmittel eingesetzt. Besonders bevorzugt kommen Chlorfluorid, Chlortrifluorid oder eine Mischung aus wenigstens zwei Fluorierungsmitteln enthaltend Chlorfluorid und/oder Chlortrifluorid zum Einsatz.

Als geeignetes Lösungsmittel für die Fluorierung der Salze der allgemeinen Formel (X) wird vorzugsweise Fluorwasserstoff, Iodpentafluorid, Dichlormethan, Chloroform oder ein Gemisch aus wenigstens zwei dieser Lösungsmittel verwendet. Fluorwasserstoff wird besonders bevorzugt als Lösungsmittel eingesetzt.

Die erfindungsgemäßen Tetrakisfluoroalkylborat-Salze der allgemeinen Formel (I) eignen sich auch zur Verwendung in festen Elektrolyten. Unter festen Elektrolyten werden dabei im Sinne der Erfindung sowohl Polymerelektrolyte verstanden, die üblicherweise ein gegebenenfalls vernetztes Polymeres und ein Leitsalz aufweisen, wie auch Gelelektrolyte, die üblicherweise neben einem gegebenenfalls vernetztem Polymeren und einem Leitsalz zusätzlich wenigstens

ein Lösungsmittel enthalten.

Ein weiterer Gegenstand der vorliegenden Erfindung ist daher auch ein Gemisch enthaltend

a) wenigstens ein Tetrakisfluoroalkylborat-Salz der allgemeinen Formel (I) ein Schließlich der zuvor ausgenommenen Salze
und

b) wenigstens ein Polymeres.

Ein Gemisch im Sinne der vorliegenden Erfindung umfaßt reine Mischungen der Komponenten a) und b), Mischungen, in denen das Salz der Komponente a) in dem Polymeren der Komponente b) eingeschlossen ist und Mischungen, in denen zwischen dem Salz der Komponente a) und dem Polymeren der Komponente b) chemische und/ oder physikalische Bindungen bestehen.

In einer bevorzugten Ausführungsform der vorliegenden Erfindung enthält das erfindungsgemäße Gemisch 5 bis 99 Gew.-% der Komponente a) und 95 bis 1 Gew.-% der Komponente b), besonders bevorzugt 60 bis 99 Gew.-% der Komponente a) und 40 bis 1 Gew.-% der Komponente b). Die angegebenen Gewichtsverhältnisse beziehen sich jeweils auf die Summe der Komponenten a) und b).

Als Komponente b) enthält das erfindungsgemäße Gemisch vorzugweise ein Homopolymeres oder Copolymeres von ungesättigten Nitrilen, vorzugsweise Acrylnitril, Vinylidenen, vorzugsweise Vinylidendifluorid, Acrylaten, vorzugsweise Methylacrylat, Methacrylaten, vorzugsweise Methylmethacrylat, zyklischen Ethern, vorzugsweise Tetrahydrofuran, Alkylenoxiden, vorzugsweise Ethylenoxid, Siloxan, Phosphazen, Alkoxysilanen oder wenigstens eine organisch modifizierte Keramik oder eine Mischung aus wenigstens zwei der vorstehend genannten Homopolymeren und/ oder Copolymeren und ggf. wenigstens einer organisch modifizierten Keramik.

Bevorzugt kommen als organisch modifizierte Keramiken anorganischorganische Hybridpolymere in Betracht, welche durch Hydrolyse und Kondensation von organisch modifizierten Silizium-Alkoxiden und anschließende Vernetzung der am anorganischen Gerüst fixierten vernetzbaren Gruppen erhalten werden. Entsprechende organisch modifizierte Keramiken werden beispielsweise unter der Bezeichnung ORMOCERE® am Markt geführt.

Besonders bevorzugt ist die Komponente b) ein Homopolymeres oder Copolymeres von Vinylidendifluorid, Acrylnitril, Methyl(meth)acrylat, Tetrahydrofuran, ganz besonders bevorzugt ein Homopolymeres oder Copolymeres von Vinylidendifluorid.

Diese Homo- und Copolymerisate des Vinylidendifluorids werden unter der Bezeichnung Kynar® und Kynarflex® von der Firma Atofina Chemicals, Inc. sowie unter der Bezeichnung Solef® von der Firma Solvay am Markt geführt.

Die erfindungsgemäß zum Einsatz kommenden Polymere können auch zumindest teilweise vernetzt sein. Die Vernetzung kann mit bekannten Vernetzungsmitteln nach üblichen, dem Fachmann bekannten Methoden erfolgen. Die Vernetzung kann auch in Gegenwart der Komponente a) und ggf. weiterer Komponenten erfolgen.

Neben den Tetrakisfluoroalkylborat-Salzen der allgemeinen Formel (I) ein Schließlich der zuvor ausgenommenen Salze sowie den Polymeren kann das erfindungsgemäße Gemisch zusätzlich ein Lösungsmittel oder ein Lösungsmittelgemisch aus zwei oder mehreren Lösungsmitteln aufweisen.

Bevorzugte Lösungsmittel sind organische Carbonate, vorzugsweise Ethylencarbonat, Propylencarbonat, Butylencarbonat, Dimethylcarbonat, Diethylcarbonat, Ethylmethylcarbonat oder Methylpropylcarbonat, organische Ester, vorzugsweise Methylformiat, Ethylformiat, Methylacetat, Ethylacetat, Methylpropionat, Ethylpropionat, Methylbutyrat, Ethylbutyrat, γ-Butyrolacton, organische Ether, vorzugsweise Diethylether, Dimethoxyethan, Diethoxyethan, organische Amide, vorzugsweise Dimethylformamid oder Dimethylacetamid, schwefelhaltige Lösungsmittel, vorzugsweise Dimethylsulfoxid, Dimethylsulfit, Diethylsulfit oder Propansulton, aprotische Lösungsmittel, vorzugsweise Acetonitril, Acrylnitril oder Aceton, oder zumindest teilweise fluorierte Derivate der vorstehend genannten Lösungsmittel oder Gemische aus wenigstens zwei dieser Lösungsmittel und/oder fluorierten Derivaten dieser Lösungsmittel.

Ebenfalls Gegenstand der vorliegenden Erfindung ist ein Verfahren zur Herstellung der erfindungsgemäßen Gemische, gemäß dem zumindest eines der vorstehend genannten Tetrakisfluoroalkylborat-Salze der allgemeinen Formel (I) ein Schließlich der zuvor ausgenommenen Salze und wenigstens ein Polymeres und gegebenfalls wenigstens ein Lösungsmittel miteinander gemischt werden.

Vorzugsweise erfolgt das Mischen dieser Komponenten bei erhöhter Temperatur, besonders bevorzugt bei 20 bis 90 °C, ganz besonders bevorzugt bei 40 bis 60 °C, wobei die Temperaturen in Abhängigkeit von den eingesetzten Komponenten variieren kann.

Ein weiterer Gegenstand der vorliegenden Erfindung ist die Verwendung wenigstens eines erfindungsgemäßen Tetrakisfluoroalkylborat-Salzes oder eines erfindungsgemäßen Gemisches in Elektrolyten, primären Batterien, sekundären Batterien, Kondensatoren, Superkondensatoren und/oder galvanischen Zellen, ggf. auch in Kombination mit weiteren, bekannten Leitsalzen und/oder Zusatzstoffen.

Die erfindungsgemäßen Tetrakisfluoroalkylborat-Salze sind ferner auch zur Polymerisation von Olefinen geeignet. Des weiteren eignen sie sich zur Herstellung von katalytisch aktiven Verbindungen, in denen die Tetrakisfluoroalkylborat-Anionen dann als Gegenion der kationischen Katalysatoren fungieren. Die Verwendung der erfindungsgemäßen Tetrakisfluoroalkylborat-Salze zur Polymerisation von Olefinen sowie zur Herstellung von Katalysatoren sind daher ebenfalls Gegenstand der vorliegenden Erfindung.

Weitere Gegenstände der Erfindung sind Elektrolyte, primäre und sekundäre Batterien, Kondensatoren, Superkondensatoren und galvanischen Zellen, die wenigstens ein erfindungsgemäßes Tetrakisfluoroalkylborat-Salz der allgemeinen Formel (I) oder ein erfindungsgemäßes Gemisch und ggf. weitere Leitsalze und/oder Zusatzstoffe enthalten. Weitere Leitsalze und Zusatzstoffe sind dem Fachmann z.B. aus Doron Auerbach, Nonaqueous Electrochemistry, Marc Dekker Inc., New York 1999; D.Linden, Handbook of Batteries, Second Edition, McGraw-Hill Inc., New York 1995 und G. Mamantov und A.I. Popov, Chemistry of Nonaqueous Solutions, Current Progress, VCH Verlagsgesellschaft, Weinheim 1994 bekannt.

Erfindungsgemäße Elektrolyte weisen bevorzugt eine Konzentration der (des) erfindungsgemäßen Tetrakisfluoroalkylborat-Salze(s) von 0,01 bis 3 mol/l, vorzugsweise 0,01 bis 2 mol/l, besonders bevorzugt 0,1 bis 1,5 mol/l auf.

Als Lösungsmittel für die erfindungsgemäßen Salze enthalten die Elektrolyte vorzugsweise organische Carbonate, vorzugsweise Ethylencarbonat, Propylencarbonat, Butylencarbonat, Dimethylcarbonat, Diethylcarbonat, Ethylmethylcarbonat oder Methylpropylcarbonat, organische Ester, vorzugsweise Methylformiat, Ethylformiat, Methylacetat, Ethylacetat, Methylpropionat, Ethylpropionat, Methylbutyrat, Ethylbutyrat, γ-Butyrolacton, organische Ether, vorzugsweise Diethylether, Dimethoxyethan, Diethoxyethan, organische Amide, vorzugsweise Dimethylformamid oder Dimethylacetamid, schwefelhaltige Lösungsmittel, vorzugsweise Dimethylsulfoxid, Dimethylsulfit, Diethylsulfit oder Propansulton, aprotische Lösungsmittel, vorzugsweise Acetonitril, Acrylnitril oder Aceton, oder zumindest teilweise fluorierte Derivate der vorstehend genannten Lösungsmittel oder Gemische aus wenigstens zwei dieser Lösungsmittel und/oder fluorierten Derivaten dieser Lösungsmittel.

Die erfindungsgemäßen Tetrakisfluoroalkylborat-Salze sowie die erfindungsgemäßen Gemische haben den Vorteil, daß sie über einen sehr langen Zeitraum in Gegenwart von Wasser keinerlei oder fast keine Anzeichen einer Zersetzung zeigen und in den meisten Lösungsmitteln oder Lösungsmittelgemischen eine gute bis sehr gute Löslichkeit aufweisen.

Desweiteren weisen sie sowohl im festen als auch im gelösten Zustand eine hohe thermische Stabilität sowie eine hohe chemische Stabilität auf. So sind die erfindungsgemäßen Salze und Gemische beispielsweise gegenüber starken Oxidationsmitteln, wie z.B. Fluor, stabil.

Diese Eigenschaften ermöglichen es, Elektrolyte, Batterien, Kondensatoren, Superkondensatoren und galvanische Zellen, die diese Leitsalze enthalten, unter extremen Bedingungen einzusetzen, wie z.B. bei hohen Temperaturen, ohne daß deren Lebensdauer und Leistung durch diese Bedingungen beeinträchtigt wird.

Desweiteren zeichnen sich die entsprechenden Batterien, Kondensatoren, Superkondensatoren und galvanischen Zellen durch eine sehr gute Spannungskonstanz, ein uneingeschränkte Funktionsfähigkeit über viele Lade- und Entladezyklen sowie durch geringe Herstellungskosten aus.

Der Einsatz der erfindungsgemäßen Tetrakisfluoroalkylborat-Salze oder der erfindungsgemäßen Gemische in großen Batterien, wie sie z.B. in Elektrostraßenfahrzeugen oder Hybridstraßenfahrzeugen verwendet werden, ist ebenfalls sehr vorteilhaft, da bei einer Beschädigung der Batterien, wie z.B. im Falle eines Unfalls, auch bei Kontakt mit Wasser, beispielsweise durch Luftfeuchtigkeit oder Löschwasser kein toxischer und stark ätzender Fluorwasserstoff gebildet wird.

Im folgenden wird die Erfindung anhand von Beispielen erläutert. Diese Beispiele dienen lediglich der Erläuterung der Erfindung und schränken den allgemeinen Erfindungsgedanken nicht ein.

**Beispiele**

Beispiel 1:

Synthese von Kaliumtetrakistrifluoromethylborat $K[B(CF_3)_4]$

1a)

85 mg (0,60 mmol) $NH_4[B(CN)_4]$ wurden in einem 250 ml PFA-(Tetrafluoroethylenperfluoropropylvinylether-Copolymer) Reaktor im Vakuum getrocknet. Anschließend wurden ca. 5 ml Fluorwasserstoff und 28,4 mmol Chlorfluorid (gasvolumetrisch abgemessen) in den Reaktor einkondensiert. Das Reaktionsgemisch wurde unter Rühren langsam auf eine Temperatur von 20 bis 25 °C erwärmt und weitere 48 Stunden bei dieser Temperatur nachgerührt. Im Vakuum wurde das Reaktionsgemisch anschließend von allen flüchtigen Bestandteilen befreit. Der so erhaltene Rückstand wurde in ca. 5 ml destilliertem Wasser aufgenommen, mit 200 mg Kaliumcarbonat neutralisiert und das Wasser anschließend wieder im Vakuum entfernt. Aus dem so erhaltenen Rückstand wurde das Kaliumtetrakistrifluoromethylborat $K[B(CF_3)_4]$

mit Diethylether extrahiert. Nach Abdestillieren des Diethylethers wurden 173 mg (0,53 mmol) $K[B(CF_3)_4]$ erhalten.

Alternativ dazu kann die Synthese von $K[B(CF_3)_4]$ auch gemäß der Vorschrift 1b) bzw. 1c) erfolgen:

1b)

1,512 g (11,4 mmol) $NH_4[B(CN)_4]$ wurden in einem 500 ml Edelstahl-Autoklaven im Vakuum getrocknet. Anschließend wurden ca. 30 bis 40 ml Fluorwasserstoff und 562 mmol Chlorfluorid (gasvolumetrisch abgemessen) in den Autoklaven einkondensiert.

Dann wurde das Reaktionsgemisch unter Rühren langsam auf eine Temperatur von 20 bis 25 °C erwärmt und weitere 48 bis 72 Stunden bei dieser Temperatur nachgerührt. Das Reaktionsgemisch wurde anschließend im Vakuum von allen flüchtigen Bestandteilen befreit. Der so erhaltene Rückstand wurde in ca. 50 ml destilliertem Wasser aufgenommen, mit 3,8g Kaliumcarbonat neutralisiert und das Wasser anschließend wieder im Vakuum entfernt. Aus dem so erhaltenen Rückstand wurde das Kaliumtetrakistrifluoromethylborat $K[B(CF_3)_4]$ mit Diethylether extrahiert. Nach Abdestillieren des Diethylethers wurden 3,4 g (11,2 mmol) $K[B(CF_3)_4]$ erhalten.

1c)

105 mg (0,79 mmol) $NH_4[B(CN)_4]$ wurden in einem 250 ml PFA-(Tetrafluoroethylenperfluoropropylvinylether-Copolymer) Reaktor im Vakuum getrocknet. Anschließend wurden ca. 5 ml Fluorwasserstoff und 11,4 mmol Chlortrifluorid (gasvolumetrisch abgemessen) in den Reaktor einkondensiert. Dann wurde das Reaktionsgemisch unter Rühren langsam auf eine Temperatur von 20 bis 25 °C erwärmt und weitere 19 Stunden bei dieser Temperatur nachgerührt. Das Reaktionsgemisch wurde anschließend im Vakuum von allen flüchtigen Bestandteilen befreit. Der so erhaltene Rückstand wurde in ca. 7 ml destilliertem Wasser aufgenommen, mit 300 mg Kaliumcarbonat neutralisiert und das Wasser anschließend wieder im Vakuum entfernt.

Aus dem so erhaltenen Rückstand wurde das Kaliumtetrakistrifluoromethylborat $K[B(CF_3)_4]$ mit Diethylether extrahiert. Nach dem Abdestillieren des Ethers wurden 209 mg (0,69 mmol) $K[B(CF_3)_4]$ erhalten.

Ein Teil des $K[B(CF_3)_4]$ wurde in deuteriertem Acetonitril gelöst (200 mg/ml, 1 mol /25 mol $CD_3CN$) und mittels $^{11}B$- und $^{19}F$-NMR-Spektroskopie charakterisiert. Die Aufnahme des $^{11}B$-NMR-Spektrums erfolgte bei einer Frequenz von 160,5 MHz, die des $^{19}F$-NMR-Spektrums bei 470,6 MHz und die des $^{13}C$-NMR-Spektrums bei 125,8 MHz. Als innerer Standard wurde für die $^{11}B$- NMR-Spektroskopie $(C_2H_5)_2OBF_3$ mit $\delta = 0$ ppm, für die $^{19}F$-NMR-Spektroskopie $CFCl_3$ mit $\delta = 0$ ppm und für die $^{13}C$-NMR-Spektroskopie Tetramethylsilan (TMS) mit $\delta = 0$ ppm eingesetzt. Die NMR-spektroskopischen Daten waren wie folgt:

$^{11}B$-NMR-Spektrum:
$\delta = -18{,}94$ ppm, $^2J(^{11}B^{19}F) = 25{,}92$ Hz, $^1J(^{11}B^{13}C) = 73{,}4$ Hz, $^1\Delta^{11}B(^{12/13}C) = 0{,}0030$ ppm bei einer Linienbreite von 0,5 Hz.

$^{19}F$-NMR-Spektrum:
$\delta = -61{,}60$ ppm, $^2J(^{19}F^{11}B) = 25{,}92$ Hz, $^2J(^{19}F^{10}B) = 8{,}68$ Hz, $^2\Delta^{19}F(^{10/11}B) = 0{,}0111$ ppm, $^1J(^{19}F^{13}C) = 304{,}3$ Hz, $^1\Delta^{19}F(^{12/13}C) = 0{,}1315$ ppm, $^3J(^{19}F^{13}C) = 3{,}9$ Hz, $^3\Delta^{19}F(^{12/13}C) = 0{,}0010$ ppm, $^4J(^{19}F^{19}F) = 5{,}8$ Hz bei einer Linienbreite von 0,4 Hz.

$^{13}C$-NMR-Spektrum:
$\delta = 132{,}9$ ppm, $^1J(^{13}C^{19}F) = 304{,}3$ Hz, $^3J(^{13}C^{19}F) = 4{,}0$ Hz, $^1J(^{13}C^{11}B) = 73{,}4$ Hz, $^1J(^{13}C^{10}B) = 24{,}6$ Hz, $^1\Delta^{13}C(^{10/11}B) = 0{,}0029$ ppm bei einer Linienbreite von 1,5 Hz.

Die Reinheit des so erhaltenen Kaliumtetrakistrifluoromethylborates betrug > 99 %. Das $K[B(CF_3)_4]$ ist sehr gut löslich in Wasser, Diethylether und Acetonitril, unlöslich dagegen in Dichlormethan, Pentan und Heptan. Nach Differential Scanning Calorimetry-Messungen ist das Salz im festen Zustand bis 320 °C ($\Delta H = -90$ J/g) stabil und weist bei -63 °C ($\Delta H = 4{,}5$ J/g) und bei - 47°C ($\Delta H = 7{,}8$ J/g) zwei Phasentransformationen auf.

Werden zur Neutralisation des jeweiligen Rohproduktes anstelle von Kaliumcarbonat, andere Carbonate oder deren Mischungen, z.B. Lithiumcarbonat, Natriumcarbonat, Rubidiumcarbonat oder Cäsiumcarbonat eingesetzt, so lassen sich analog die entsprechenden Lithium-, Natrium-, Rubidium oder Cäsiumtetrakistrifluoromethylboratsalze erhalten, wobei in dem entsprechenden Lithium- bzw. Natriumsalz Lösungsmittelmoleküle gebunden sind, die durch langsames Erwärmen des jeweiligen Salzes sukzessive entfernt werden können.

Beispiel 2:

Synthese von $[Li(THF)_X][B(CF_3)_4]$

173 mg $K[B(CF_3)_4]$ (0.57 mmol) und 24 mg Lithiumchlorid wurden in einem 50 ml Glas-Kolben mit Polytetrafluoroethylen-Ventil im Vakuum getrocknet. Anschließend wurden ca. 10 ml Tetrahydrofuran in den Kolben einkondensiert und das Reaktionsgemisch eine Stunde bei einer Temperatur von 20 bis 25 °C gerührt. Hierbei bildete sich ein Niederschlag von Kaliumchlorid, der anschließend abfiltriert wurde. Die so erhaltene Lösung wurde bei einer Temperatur von 20 bis 25 °C im Vakuum vollständig eingeengt. Nach dem Entfernen des Tetrahydrofurans wurden 300 mg $[Li(THF)_X][B(CF_3)_4]$ erhalten.
Lithiumtetrakistrifluoromethylborat ist im festen Zustand bis 168 °C stabil ($\Delta H$ = -260 J/g). Es ist sehr gut löslich in Wasser, Tetrahydrofuran, Acetonitril, Methanol und Aceton. Die Tetrahydrofuran-Solvatmoleküle werden bis 140 °C stufenweise entfernt (97 °C, $\Delta H$ = 7 g/J, 130 °C, $\Delta H$ = 4 J/g).

Beispiel 3:

Synthese von $Li[B(CF_3)_4]$

4,233 g (12,99 mmol) $K[B(CF_3)_4]$ und 1,225 g (13,07 mmol) $Li[BF_4]$ wurden in 13,052 g Lösungsmittelgemisch aus Ethylencarbonat, Dimethylcarbonat und Diethylcarbonat im Verhältnis 2 : 1 : 2 gegeben. Hierbei bildete sich ein Niederschlag von $K[BF_4]$, der durch Filtration abgetrennt wurde. Die so erhaltene Lösung von $Li[B(CF_3)_4]$ (14,522 g, 11,7 ml) wies eine Konzentration des Salzes von 22,6 Gew.-% bzw. 0,96 mol/l auf.

Beispiel 4:

Synthese von 1-Ethyl-3-methylimidazoliumtetrakistrifluormethylborat

Äquimolare Mengen von Kaliumtetrakistrifluormethylborat und 1-Ethyl-3-methylimidazoliumchlorid wurden bei einer Temperatur von 20 bis 25 °C in Acetonitril suspendiert. Anschließend wurde dieses Gemisch 10 Stunden bei dieser Temperatur gerührt und unter Kühlung über eine Glasfritte vakuumfiltriert, um das gebildete Kaliumchlorid vollständig zu entfernen. Das Lösungsmittel wurde im Vakuum abdestilliert und das so erhalten Produkt im Vakuum getrocknet.

Beispiel 5:

Vergleich der spezifischen ionischen Leitfähigkeit von $Li[B(CF_3)_4]$ und $LiPF_6$:
Es wurde jeweils eine Lösung des entsprechenden Salzes in einer Mischung aus Ethylencarbonat, Diethylcarbonat und Dimethylcarbonat im Volumenverhältnis 2:1:2 angesetzt und bei einer Temperatur von 25 °C gemessen.
Die Leitfähigkeitsmessung wurde mit Hilfe eines Knick Konduktometers 703 und einer Knick 4-Pol-Messzelle mit Hüllrohr durchgeführt. Die Thermostatierung erfolgte im Klimaschrank TI 4, die Temperaturkontrolle erfolgte mit Hilfe eines Pt 100 Widerstandsthermometers.
Die entsprechenden Konzentrationen sowie die entsprechenden ionischen Leitfähigkeiten sind in der nachfolgenden Tabelle 1 angegeben:

Tabelle 1:

| | $Li[B(CF_3)_4]$ | $LiPF_6$ |
|---|---|---|
| Konzentration der Lösung in mol/l | 0,96 | 1 |
| ionische Leitfähigkeit in mS/cm | 10,1 | 9,6 |

Das erfindungsgemäße $Li[B(CF_3)_4]$-Salz zeigt eine im Vergleich zu $LiPF_6$ verbesserte spezifische ionische Leitfähigkeit.

Beispiel 6:

Untersuchung der elektrochemischen Stabilität $Li[B(CF_3)_4]$:
In einer Messzelle mit Platinarbeitselektrode, Lithiumgegenelektrode und Lithiumreferenzelektrode wurden für eine 0,96 molare Lösung von $Li[B(CF_3)_4]$ in Ethylencarbonat, Diethylcarbonat und Dimethylcarbonat (Volumenverhältnis

von 2:1:2) drei aufeinanderfolgende Zyklovoltammogramme aufgenommen. Dazu wurde ausgehend vom Ruhepotential das Potential zunächst mit einer Vorschubgeschwindigkeit von 10 mV/s auf 6,0 Volt gegen das Potential von $Li/Li^+$ erhöht und dann wieder zurück auf das Ruhepotential abgesenkt. Die so erhaltenen Zyklovoltammogramme zeigten keine Anzeichen einer Zersetzung des Elektrolyten.

## Patentansprüche

**1.** Tetrakisfluoroalkylborat-Salzen der allgemeinen Formel (I),

$$M^{n+} ([BR_4]^-)_n \qquad (I)$$

worin
$M^{n+}$ ein einwertiges, zweiwertiges oder dreiwertiges Kation ist,
die Liganden R jeweils gleich und geradkettig oder verzweigt sind und für $(C_xF_{2x+1})$ mit $1 \leq x \leq 8$ stehen
und n = 1, 2 oder 3 ist;
wobei Tetrakisfluoroalkylborat-Salze ausgenommen sind, worin $M^{n+}$ für ein Alkalimetall-Kation oder für ein Ammonium-Ion der allgemeinen Formel $NR'_4$ steht, wobei R' jeweils unabhängig voneinander für H, Alkyl-, Alkenyl- oder Aryl-Gruppen steht, wenn die Liganden R jeweils für $(C_xF_{2x+1})$ mit $1 \leq x \leq 4$ stehen.

**2.** Tetrakisfluoroalkylborat-Salze gemäß Anspruch 1, **dadurch gekennzeichnet, daß** das Kation $M^{n+}$ ein Magnesium- oder Aluminium-Kation ist.

**3.** Tetrakisfluoroalkylborat-Salze gemäß Anspruch 1, **dadurch gekennzeichnet, daß** $M^{n+}$ ein organisches Kation, vorzugsweise
ein Nitrosyl-Kation, ein Nitryl-Kation oder ein Kation der allgemeinen Formel $[N(R^7)_4]^+$, $[P(N(R^7)_2)_kR_{4-k}]^+$ mit $0 \leq k \leq 4$ oder $[C(N(R^7)_2)_3]^+$ ist, wobei die Reste $R^7$, jeweils gleich oder verschieden, für
H,
$C_oF_{2o+1-p-q}H_pA_q$ oder
A stehen,
worin
$1 \leq o \leq 10$,
$0 \leq p \leq 2o+1$
$0 \leq q \leq 2o+1$ und
A jeweils einen gegebenenfalls Heteroatome aufweisenden aromatischen Rest oder einen vorzugsweise 5- oder 6-gliedrigen Cycloalkyl-Rest bedeutet.

**4.** Tetrakisfluoroalkylborat-Salze gemäß Anspruch 3, **dadurch gekennzeichnet,**
**daß** 1 $1 \leq o \leq 6$, $0 \leq p \leq 2o+1$, $0 \leq q \leq 2o+1$ sind und
A jeweils einen gegebenenfalls Heteroatome aufweisenden aromatischen Rest oder einen vorzugsweise 5- oder 6-gliedrigen Cycloalkyl-Rest bedeutet.

**5.** Tetrakisfluoroalkylborat-Salze gemäß Anspruch 3 oder 4, **dadurch gekennzeichnet, daß** A jeweils einen 5- oder 6-gliedrigen, gegebenenfalls Stickstoff- und/oder Schwefel- und/oder Sauerstoffatome aufweisenden aromatischen Rest oder einen vorzugsweise 5- oder 6-gliedrigen Cycloalkyl-Rest, vorzugsweise einen Phenyl- oder Pyridin-Rest bedeutet.

**6.** Tetrakisfluoroalkylborat-Salze gemäß Anspruch 1, **dadurch gekennzeichnet, daß** $M^{n+}$ ein heteroaromatisches Kation ausgewählt aus der Gruppe der heteroaromatischen Kationen der allgemeinen Formel (II) bis (IX)

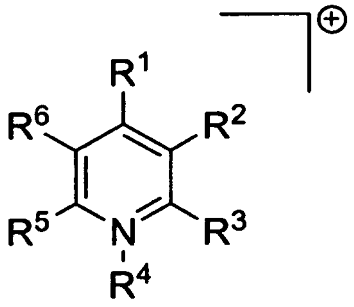

(II)

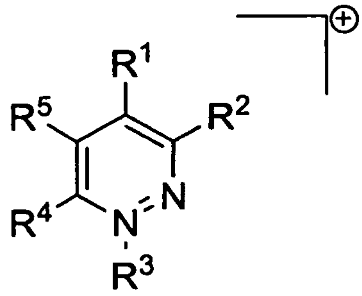

(III)

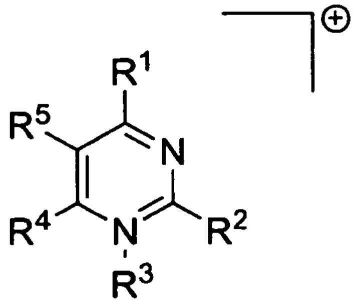

(IV)

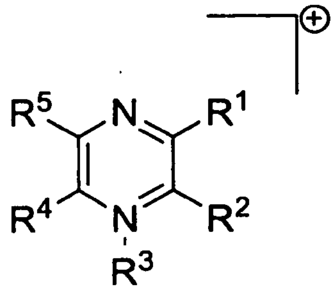

(V)

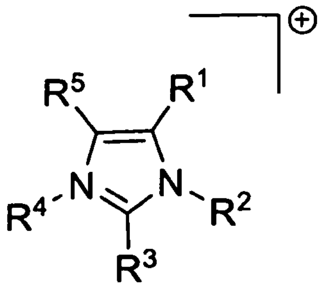

(VI)

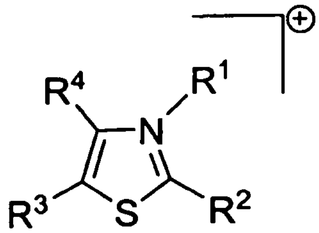

(VII)

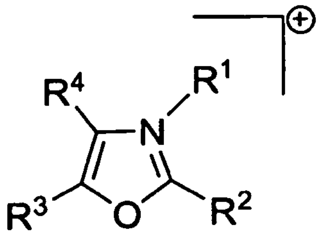

(VIII)

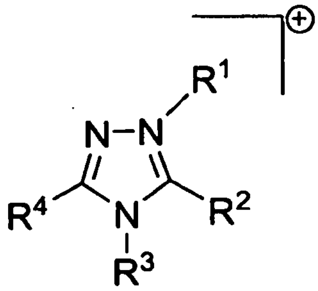

(IX)

ist, worin die Reste $R^1$ bis $R^6$, die jeweils gleich oder verschieden, ggf. auch zwei der Reste $R^1$ bis $R^6$ gemeinsam, einen H-Rest, einen Halogen-Rest, vorzugsweise einen Fluor-Rest, oder einen $C_{1-8}$-Alkyl-Rest bedeuten, der gegebenenfalls mit
F, Cl, $N(C_aF_{(2a+1-b)}H_b)_2$, $O(C_aF_{(2a+1-b)}H_b)$, $SO_2(C_aF_{(2a+1-b)}H_b)$ oder $CaF_{(2a+1-b)}H_b$ substituiert sein kann, worin $1 \leq a \leq 6$ und $0 \leq b \leq 2a+1$ bedeuten.

**7.** Tetrakisfluoroalkylborat-Salze gemäß einem der Ansprüche 1 bis 6, **dadurch gekennzeichnet, daß** die Liganden R jeweils gleich sind und für $(C_xF_{2x}+1)$ mit x = 1 oder 2 stehen.

**8.** Tetrakisfluoroalkylborat-Salze gemäß einem der Ansprüche 1 bis 7, **dadurch gekennzeichnet, daß** die Liganden R jeweils gleich sind und für einen $CF_3$-Rest stehen.

**9.** Verfahren zur Herstellung von Tetrakisfluoroalkylborat-Salzen gemäß Anspruch 8, **dadurch gekennzeichnet, daß** wenigstens eine Verbindung der allgemeinen Formel (X),

$$M^{n+} ([B(CN)_4]^-)_n \qquad (X)$$

worin $M^{n+}$ und n die Bedeutung gemäß den Ansprüchen 1 bis 8 einschließlich der vorstehend ausgenommenen Bedeutung haben,
durch Umsetzung mit wenigstens einem Fluorierungsmittel in wenigstens einem Lösungsmittel fluoriert und die so erhaltene fluorierte Verbindung der allgemeinen Formel (I) gemäß Anspruch 1 nach den üblichen Methoden gereinigt und isoliert wird.

**10.** Verfahren nach Anspruch 9, **dadurch gekennzeichnet, daß** die Umsetzung mit dem Fluorierungsmittel bei einer Temperatur im Bereich von -80 bis +20 °C, vorzugsweise im Bereich von -60 bis 0 °C durchgeführt wird.

**11.** Verfahren gemäß Anspruch 9 oder 10, **dadurch gekennzeichnet, daß** als Fluorierungsmittel Fluor, Chlorfluorid, Chlortrifluorid, Chlorpentafluorid, Bromtrifluorid, Brompentafluorid oder ein Gemisch aus wenigstens zwei dieser Fluorierungsmittel, vorzugsweise Chlorfluorid oder Chlortrifluorid oder eine Mischung aus wenigstens zwei Fluorierungsmitteln enthaltend Chlorfluorid und/oder Chlortrifluorid eingesetzt wird.

**12.** Verfahren gemäß einem der Ansprüche 9 bis 11, **dadurch gekennzeichnet, daß** als Lösungsmittel Fluorwasserstoff, Iodpentafluorid, Dichlormethan, Chloroform oder ein Gemisch aus wenigstens zwei dieser Lösungsmittel, vorzugsweise Fluorwasserstoff, eingesetzt wird.

**13.** Gemisch enthaltend

a) wenigstens ein Tetrakisfluoroalkylborat-Salz der allgemeinen Formel (I) gemäß den Ansprüchen 1 bis 8 einschließlich der vorstehend ausgenommenen Salze
und
b) wenigstens ein Polymeres.

**14.** Gemisch gemäß Anspruch 13 **dadurch gekennzeichnet, daß** es 5 bis 99 Gew.% der Komponente a) und 95 bis 1 Gew.-% der Komponente b), vorzugsweise 60 bis 99 Gew.-% der Komponente a) und 40 bis 1 Gew.-% der Komponente b), jeweils bezogen auf die Summe der Komponenten a) und b), enthält.

**15.** Gemisch gemäß Anspruch 13 oder 14, **dadurch gekennzeichnet, daß** die Komponente b) ein Homopolymeres oder Copolymeres von ungesättigten Nitrilen, vorzugsweise Acrylnitril, Vinylidenen, vorzugsweise Vinylidendifluorid, Acrylaten, vorzugsweise Methylacrylat, Methacrylaten, vorzugsweise Methylmethacrylat, zyklischen Ethern, vorzugsweise Tetrahydrofuran, Alkylenoxiden, vorzugsweise Ethylenoxid, Siloxan, Phosphazen, Alkoxysilanen oder eine organisch modifizierte Keramik oder eine Mischung aus wenigstens zwei der vorstehend genannten Homopolymeren und/oder Copolymeren und ggf. wenigstens einer organisch modifizierten Keramik ist.

**16.** Gemisch gemäß Anspruch 15, **dadurch gekennzeichnet, daß** die Komponente b) ein Homopolymeres oder Copolymeres von Vinylidendifluorid, Acrylnitril, Methyl(meth)acrylat, Tetrahydrofuran, vorzugsweise ein Homopolymeres oder Copolymeres von Vinylidendifluorid ist.

**17.** Gemisch gemäß einem der Ansprüche 13 bis 16, **dadurch gekennzeichnet, daß** das Polymere zumindest teilweise vernetzt ist.

**18.** Gemisch gemäß einem der Ansprüche 13 bis 17, **dadurch gekennzeichnet, daß** es zusätzlich wenigstens ein Lösungsmittel enthält.

**19.** Gemisch gemäß Anspruch 18, **dadurch gekennzeichnet, daß** als Lösungsmittel organische Carbonate, vorzugsweise Ethylencarbonat, Propylencarbonat, Butylencarbonat, Dimethylcarbonat, Diethylcarbonat, Ethylmethylcarbonat oder Methylpropylcarbonat, organische Ester, vorzugsweise Methylformiat, Ethylformiat, Methylacetat, Ethylacetat, Methylpropionat, Ethylpropionat, Methylbutyrat, Ethylbutyrat, γ-Butyrolacton, organische Ether, vorzugsweise Diethylether, Dimethoxyethan, Diethoxyethan, organische Amide, vorzugsweise Dimethylformamid oder Dimethylacetamid, schwefelhaltige Lösungsmittel, vorzugsweise Dimethylsulfoxid, Dimethylsulfit, Diethylsulfit oder Propansulton, aprotische Lösungsmittel, vorzugsweise Acetonitril, Acrylnitril oder Aceton, oder zumindest teilweise fluorierte Derivate der vorstehend genannten Lösungsmittel oder Gemische aus wenigstens zwei dieser Lösungsmittel und/oder fluorierten Derivaten dieser Lösungsmittel vorliegen.

**20.** Verfahren zur Herstellung eines Gemisches gemäß einem der Ansprüche 13 bis 19, **dadurch gekennzeichnet, daß** man wenigstens ein Tetrakisfluoroalkylborat-Salz der allgemeinen Formel (I) gemäß einem der Ansprüche 1 bis 8 einschließlich der vorstehend ausgenommenen Salze und wenigstens ein Polymeres und gegebenenfalls wenigstens ein Lösungsmittel mischt.

**21.** Verfahren gemäß Anspruch 20, **dadurch gekennzeichnet, daß** das Mischen bei erhöhter Temperatur, vorzugsweise bei 20 bis 90 °C, besonders bevorzugt bei 40 bis 60 °C erfolgt.

**22.** Verwendung wenigstens eines Tetrakisfluoroalkylborat-Salzes der allgemeinen Formel (I) gemäß einem der Ansprüche 1 bis 8 oder wenigstens eines Gemisches gemäß einem der Ansprüche 13 bis 19 in Elektrolyten, primären Batterien, sekundären Batterien, Kondensatoren, Superkondensatoren oder galvanischen Zellen, gegebenenfalls auch in Kombination mit weiteren Leitsalzen und/oder Zusatzstoffen.

**23.** Elektrolyte enthaltend wenigstens ein Tetrakisfluoroalkylborat-Salz der allgemeinen Formel (I) gemäß einem der Ansprüche 1 bis 8 oder wenigstens ein Gemisch gemäß einem der Ansprüche 13 bis 19.

**24.** Elektrolyte gemäß Anspruch 23, **dadurch gekennzeichnet, daß** die Konzentration des/der Tetrakisfluoroalkylborat-Salze(s) in dem Elektrolyten 0,01 bis 3 mol/l, vorzugsweise 0,01 bis 2 mol/l, besonders bevorzugt 0,1 bis 1,5 mol/l beträgt.

**25.** Primäre Batterien enthaltend wenigstens ein Tetrakisfluoroalkylborat-Salz der allgemeinen Formel (I) gemäß einem der Ansprüche 1 bis 8 oder wenigstens ein Gemisch gemäß einem der Ansprüche 13 bis 19.

**26.** Sekundäre Batterien enthaltend wenigstens ein Tetrakisfluoroalkylborat-Salz der allgemeinen Formel (I) gemäß einem der Ansprüche 1 bis 8 oder wenigstens ein Gemisch gemäß einem der Ansprüche 13 bis 19.

**27.** Kondensatoren enthaltend wenigstens ein Tetrakisfluoroalkylborat-Salz der allgemeinen Formel (I) gemäß einem

der Ansprüche 1 bis 8 oder wenigstens ein Gemisch gemäß einem der Ansprüche 13 bis 19.

**28.** Superkondensatoren enthaltend wenigstens ein Tetrakisfluoroalkylborat-Salz der allgemeinen Formel (I) gemäß einem der Ansprüche 1 bis 8 oder wenigstens ein Gemisch gemäß einem der Ansprüche 13 bis 19.

**29.** Galvanische Zellen enthaltend wenigstens ein Tetrakisfluoroalkylborat-Salz der allgemeinen Formel (I) gemäß einem der Ansprüche 1 bis 8 oder wenigstens ein Gemisch gemäß einem der Ansprüche 13 bis 19.

## Claims

**1.** Tetrakisfluoroalkylborate salts of the general formula (I)

$$M^{n+} ([BR_4]^-)_n \qquad (I)$$

in which
$M^{n+}$ is a monovalent, divalent or trivalent cation,
the ligands R are in each case identical and straight-chain or branched and stand for $(C_xF_{2x+1})$, where $1 \leq x \leq 8$ and n = 1, 2 or 3;
where tetrakisfluoroalkylborate salts in which $M^{n+}$ stands for an alkali metal cation or for an ammonium ion of the general formula $NR'_4$, where R' in each case, independently of one another, stands for H, alkyl, alkenyl or aryl groups if the ligands R each stand for $(C_xF_{2x+1})$, where $1 \leq x \leq 4$, are excluded.

**2.** Tetrakisfluoroalkylborate salts according to Claim 1, **characterised in that** the cation $M^{n+}$ is a magnesium or aluminium cation.

**3.** Tetrakisfluoroalkylborate salts according to Claim 1, **characterised in that** $M^{n+}$ is an organic cation, preferably a nitrosyl cation, a nitryl cation or a cation of the general formula $[N(R^7)_4]^+$, $[P(N(R^7)_2)_kR_{4-k}]^+$, where $0 \leq k \leq 4$, or $[C(N(R^7)_2)_3]^+$, where the radicals $R^7$, in each case identically or differently, stand for
H,
$C_oF_{2o+1-p-q}H_pA_q$ or
A,
in which
$1 \leq o \leq 10$,
$0 \leq p \leq 2o+1$,
$0 \leq q \leq 2o+1$ and
A in each case denotes an aromatic radical, optionally containing heteroatoms, or a cycloalkyl radical, preferably having 5 or 6 members.

**4.** Tetrakisfluoroalkylborate salts according to Claim 3, **characterised in that** $1 \leq o \leq 6$, $0 \leq p \leq 2o+1$, $0 \leq q \leq 2o+1$ and A in each case denotes an aromatic radical, optionally containing heteroatoms, or a cycloalkyl radical, preferably having 5 or 6 members.

**5.** Tetrakisfluoroalkylborate salts according to Claim 3 or 4, **characterised in that** A in each case denotes a 5- or 6-membered aromatic radical, optionally containing nitrogen and/or sulfur and/or oxygen atoms, or a cycloalkyl radical, preferably having 5 or 6 members, preferably a phenyl or pyridine radical.

**6.** Tetrakisfluoroalkylborate salts according to Claim 1, **characterised in that** $M^{n+}$ is a heteroaromatic cation selected from the group of the heteroaromatic cations of the general formulae (II) to (IX)

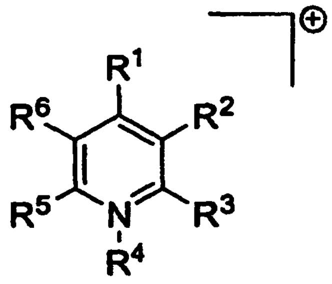

(II)

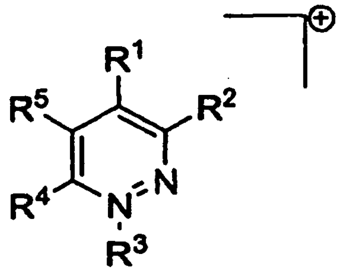

(III)

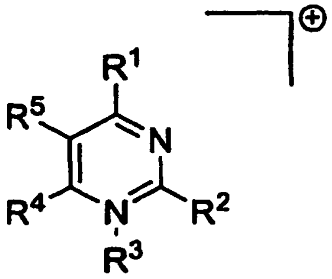

(IV)

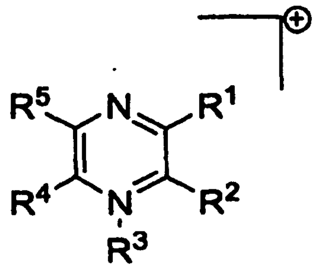

(V)

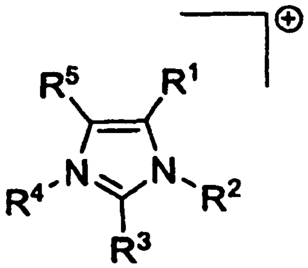

(VI)

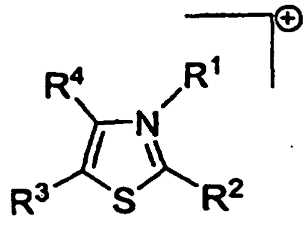

(VII)

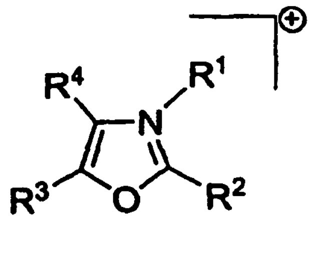

(VIII)

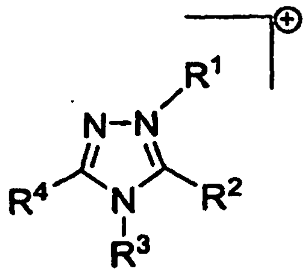

(IX)

in which the radicals $R^1$ to $R^6$, in each case identically or differently, optionally also two of the radicals $R^1$ to $R^6$ together, denote an H radical, a halogen radical, preferably a fluorine radical, or a $C_{1-8}$-alkyl radical, which may optionally be substituted by

F, Cl, $N(C_aF_{(2a+1-b)}H_b)_2$, $O(C_aF_{(2a+1-b)}H_b)$, $SO_2(C_aF_{(2a+1-b)}H_b)$ or $C_aF_{(2a+1-b)}H_b$, in which $1 \leq a \leq 6$ and $0 \leq b \leq 2a+1$.

7. Tetrakisfluoroalkylborate salts according to one of Claims 1 to 6, **characterised in that** the ligands R are in each case identical and stand for $(C_xF_{2x+1})$, where x = 1 or 2.

8. Tetrakisfluoroalkylborate salts according to one of Claims 1 to 7, **characterised in that** the ligands R are in each case identical and stand for a $CF_3$ radical.

9. Process for the preparation of tetrakisfluoroalkylborate salts according to Claim 8, **characterised in that** at least one compound of the general formula (X)

$$M^{n+} ([B(CN)_4]^-)_n \qquad (X)$$

in which $M^{n+}$ and n have the meaning according to Claims 1 to 8, including the meaning excluded above,
is fluorinated by reaction with at least one fluorinating agent in at least one solvent, and the fluorinated compound of the general formula (I) according to Claim 1 obtained in this way is purified and isolated by conventional methods.

10. Process according to Claim 9, **characterised in that** the reaction with the fluorinating agent is carried out at a temperature in the range from -80 to +20°C, preferably in the range from -60 to 0°C.

11. Process according to Claim 9 or 10, **characterised in that** the fluorinating agent employed is fluorine, chlorine fluoride, chlorine trifluoride, chlorine pentafluoride, bromine trifluoride, bromine pentafluoride or a mixture of at least two of these fluorinating agents, preferably chlorine fluoride or chlorine trifluoride, or a mixture of at least two fluorinating agents comprising chlorine fluoride and/or chlorine trifluoride.

12. Process according to one of Claims 9 to 11, **characterised in that** the solvent employed is hydrogen fluoride, iodine pentafluoride, dichloromethane, chloroform or a mixture of at least two of these solvents, preferably hydrogen fluoride.

13. Mixture comprising

a) at least one tetrakisfluoroalkylborate salt of the general formula (I) according to Claims 1 to 8, including the salts excluded above,
and
b) at least one polymer.

14. Mixture according to Claim 13, **characterised in that** it comprises 5 to 99% by weight of component a) and 95 to 1% by weight of component b), preferably 60 to 99% by weight of component a) and 40 to 1% by weight of component b), in each case based on the sum of components a) and b).

**15.** Mixture according to Claim 13 or 14, **characterised in that** component b) is a homopolymer or copolymer of unsaturated nitriles, preferably acrylonitrile, vinylidenes, preferably vinylidene difluoride, acrylates, preferably methyl acrylate, methacrylates, preferably methyl methacrylate, cyclic ethers, preferably tetrahydrofuran, alkylene oxides, preferably ethylene oxide, siloxane, phosphazene, alkoxysilanes, or an organically modified ceramic, or a mixture of at least two of the above-mentioned homopolymers and/or copolymers and optionally at least one organically modified ceramic.

**16.** Mixture according to Claim 15, **characterised in that** component b) is a homopolymer or copolymer of vinylidene difluoride, acrylonitrile, methyl (meth)acrylate, tetrahydrofuran, preferably a homopolymer or copolymer of vinylidene difluoride.

**17.** Mixture according to one of Claims 13 to 16, **characterised in that** the polymer is at least partially crosslinked.

**18.** Mixture according to one of Claims 13 to 17, **characterised in that** it additionally comprises at least one solvent.

**19.** Mixture according to Claim 18, **characterised in that** the solvents present are organic carbonates, preferably ethylene carbonate, propylene carbonate, butylene carbonate, dimethyl carbonate, diethyl carbonate, ethyl methyl carbonate or methyl propyl carbonate, organic esters, preferably methyl formate, ethyl formate, methyl acetate, ethyl acetate, methyl propionate, ethyl propionate, methyl butyrate, ethyl butyrate, γ-butyrolactone, organic ethers, preferably diethyl ether, dimethoxyethane, diethoxyethane, organic amides, preferably dimethylformamide or dimethylacetamide, sulfur-containing solvents, preferably dimethyl sulfoxide, dimethyl sulfite, diethyl sulfite or propane sultone, aprotic solvents, preferably acetonitrile, acrylonitrile or acetone, or at least partially fluorinated derivatives of the above-mentioned solvents, or mixtures of at least two of these solvents and/or fluorinated derivatives of these solvents.

**20.** Process for the preparation of a mixture according to one of Claims 13 to 19, **characterised in that** at least one tetrakisfluoroalkylborate salt of the general formula (I) according to one of Claims 1 to 8, including the salts excluded above, and at least one polymer and optionally at least one solvent are mixed.

**21.** Process according to Claim 20, **characterised in that** the mixing is carried out at elevated temperature, preferably at 20 to 90°C, particularly preferably at 40 to 60°C.

**22.** Use of at least one tetrakisfluoroalkylborate of the general formula (I) according to one of Claims 1 to 8 or at least one mixture according to one of Claims 13 to 19 in electrolytes, primary batteries, secondary batteries, capacitors, supercapacitors or galvanic cells, optionally also in combination with other conductive salts and/or additives.

**23.** Electrolytes comprising at least one tetrakisfluoroalkylborate salt of the general formula (I) according to one of Claims 1 to 8 or at least one mixture according to one of Claims 13 to 19.

**24.** Electrolytes according to Claim 23, **characterised in that** the concentration of the tetrakisfluoroalkylborate salt(s) in the electrolyte is 0.01 to 3 mol/l, preferably 0.01 to 2 mol/l, particularly preferably 0.1 to 1.5 mol/l.

**25.** Primary batteries containing at least one tetrakisfluoroalkylborate salt of the general formula (I) according to one of Claims 1 to 8 or at least one mixture according to one of Claims 13 to 19.

**26.** Secondary batteries containing at least one tetrakisfluoroalkylborate salt of the general formula (I) according to one of Claims 1 to 8 or at least one mixture according to one of Claims 13 to 19.

**27.** Capacitors containing at least one tetrakisfluoroalkylborate salt of the general formula (I) according to one of Claims 1 to 8 or at least one mixture according to one of Claims 13 to 19.

**28.** Supercapacitors containing at least one tetrakisfluoroalkylborate salt of the general formula (I) according to one of Claims 1 to 8 or at least one mixture according to one of Claims 13 to 19.

**29.** Galvanic cells containing at least one tetrakisfluoroalkylborate salt of the general formula (I) according to one of Claims 1 to 8 or at least one mixture according to one of Claims 13 to 19.

## Revendications

1. Sels de tétrakisfluoroalkylborate de formule générale (I)

$$M^{n+} ([BR_4]^-)_n \qquad (I)$$

dans laquelle
$M^{n+}$ est un cation monovalent, divalent ou trivalent,
les ligands R sont dans chaque cas identiques et à chaîne linéaire ou ramifiée et représentent ($C_xF_{2x+1}$), où $1 \leq x \leq 8$ et n = 1, 2 ou 3;
où les sels de tétrakisfluoroalkylborate dans lesquels $M^{n+}$ représente un cation de métal alcalin ou un ion ammonium de formule générale $NR'_4$, où R' dans chaque cas, indépendamment l'un de l'autre, représente H, des groupements alkyle, alcényle ou aryle si les ligands R représentent chacun ($C_xF_{2x+1}$), où $1 \leq x \leq 4$, sont exclus.

2. Sels de tétrakisfluoroalkylborate selon la revendication 1, **caractérisés en ce que** le cation $M^{n+}$ est un cation de magnésium ou d'aluminium.

3. Sels de tétrakisfluoroalkylborate selon la revendication 1, **caractérisés en ce que** $M^{n+}$ est un cation organique, préférablement un cation nitrosyle, un cation nitryle ou un cation de formule générale $[N(R^7)_4]^+$, $[P(N(R^7)_2)_kR_{4-k}]^+$, où $0 \leq k \leq 4$, ou $[C(N(R^7)_2)_3]^+$, où les radicaux $R^7$, dans chaque cas de manière identique ou différente, représentent
H,
$C_oF_{2o+1-p-q}H_pA_q$ ou
A,
où
$1 \leq o \leq 10$,
$0 \leq p \leq 2o+1$,
$0 \leq q \leq 2o+1$ et
A dans chaque cas désigne un radical aromatique, contenant éventuellement des hétéroatomes, ou un radical cycloalkyle, ayant de préférence 5 ou 6 chaînons.

4. Sels de tétrakisfluoroalkylborate selon la revendication 3, **caractérisés en ce que** $1 \leq o \leq 6$, $0 \leq p \leq 2o+1$, $0 \leq q \leq 2o+1$ et A dans chaque cas désigne un radical aromatique, contenant éventuellement des hétéroatomes, ou un radical cycloalkyle, ayant de préférence 5 ou 6 chaînons.

5. Sels de tétrakisfluoroalkylborate selon la revendication 3 ou 4, **caractérisés en ce que** A dans chaque cas désigne un radical aromatique de 5 ou 6 chaînons, contenant éventuellement des atomes d'azote et/ou de soufre et/ou d'oxygène, ou un radical cycloalkyle, ayant de préférence 5 ou 6 chaînons, préférablement un radical phényle ou pyridine.

6. Sels de tétrakisfluoroalkylborate selon la revendication 1, **caractérisés en ce que** $M^{n+}$ est un cation hétéroaromatique choisi parmi le groupe des cations hétéroaromatiques de formules générales (II) à (IX)

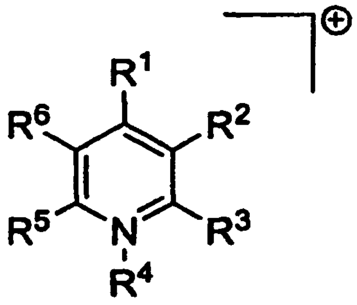

(II)

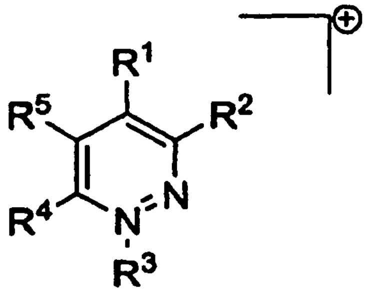

(III)

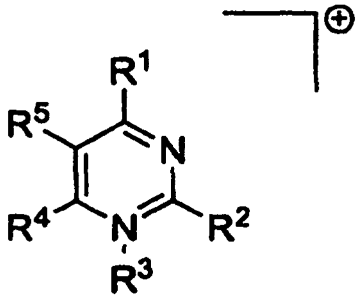

(IV)

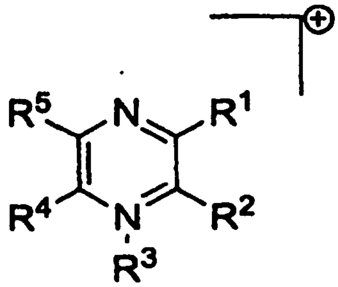

(V)

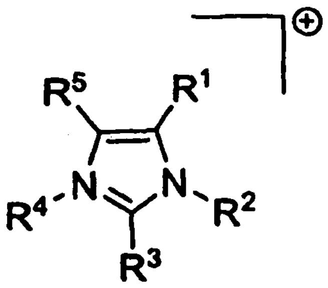

(VI)

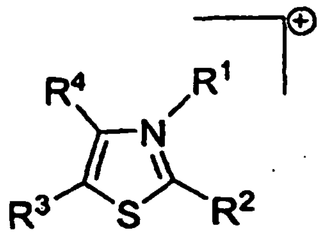

(VII)

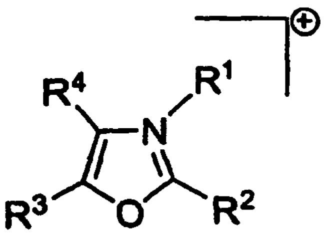

(VIII)

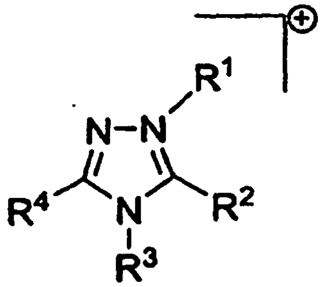

(IX)

dans lesquelles les radicaux $R^1$ à $R^6$, dans chaque cas de manière identique ou différente, éventuellement aussi deux parmi les radicaux $R^1$ à $R^6$ ensemble, désignent un radical H, un radical halogène, préférablement un radical fluor, ou un radical $C_{1-8}$-alkyle, pouvant éventuellement être substitué par F, Cl, $N(C_aF_{(2a+1-b)}H_b)_2$, $O(C_aF_{(2a+1-b)}H_b)$, $SO_2(C_aF_{(2a+1-b)}H_b)$ ou $C_aF_{(2a+1-b)}H_b$, dans lesquels $1 \leq a \leq 6$ et $0 \leq b \leq 2a+1$.

7. Sels de tétrakisfluoroalkylborate selon l'une des revendications 1 à 6, **caractérisés en ce que** les ligands R sont dans chaque cas identiques et représentent $(C_xF_{2x+1})$, où x = 1 ou 2.

8. Sels de tétrakisfluoroalkylborate selon l'une des revendications 1 à 7, **caractérisés en ce que** les ligands R sont dans chaque cas identiques et représentent un radical $CF_3$.

9. Procédé de préparation de sels de tétrakisfluoroalkylborate selon la revendication 8, **caractérisé en ce qu'**au moins un composé de formule générale (X)

$$M^{n+} ([B(CN)_4]^-)_n \qquad (X)$$

dans laquelle $M^{n+}$ et n revêtent la signification selon les revendications 1 à 8, y compris la signification exclue ci-dessus,
est fluoré par réaction avec au moins un agent de fluoration dans au moins un solvant, et le composé fluoré de formule générale (I) selon la revendication 1 obtenu de cette façon est purifié et isolé par des méthodes classiques.

10. Procédé selon la revendication 9, **caractérisé en ce que** la réaction avec l'agent de fluoration est effectuée à une température dans la plage allant de -80 à +20°C, préférablement dans la plage allant de -60 à 0°C.

11. Procédé selon la revendication 9 ou 10, **caractérisé en ce que** l'agent de fluoration employé est le fluor, le fluorure de chlore, le trifluorure de chlore, le pentafluorure de chlore, le trifluorure de brome, le pentafluorure de brome ou un mélange d'au moins deux parmi ces agents de fluoration, préférablement le fluorure de chlore ou le trifluorure de chlore, ou un mélange d'au moins deux agents de fluoration comprenant le fluorure de chlore et/ou le trifluorure de chlore.

12. Procédé selon l'une des revendications 9 à 11, **caractérisé en ce que** le solvant employé est le fluorure d'hydrogène, le pentafluorure d'iode, le dichlorométhane, le chloroforme ou un mélange d'au moins deux parmi ces solvants, préférablement le fluorure d'hydrogène.

13. Mélange comprenant

a) au moins un sel de tétrakisfluoroalkylborate de formule générale (I) selon les revendications 1 à 8, y compris les sels exclus ci-dessus,
et
b) au moins un polymère.

**14.** Mélange selon la revendication 13, **caractérisé en ce qu'**il comprend de 5 à 99% en poids de composant a) et de 95 à 1% en poids de composant b), préférablement de 60 à 99% en poids de composant a) et de 40 à 1% en poids de composant b), dans chaque cas sur la base de la somme des composants a) et b).

**15.** Mélange selon la revendication 13 ou 14, **caractérisé en ce que** le composant b) est un homopolymère ou copolymère de nitriles insaturés, préférablement d'acrylonitrile, de vinylidènes, préférablement de difluorure de vinylidène, d'acrylates, préférablement d'acrylate de méthyle, de méthacrylates, préférablement de méthacrylate de méthyle, d'éthers cycliques, préférablement de tétrahydrofurane, d'oxydes d'alkylène, préférablement d'oxyde d'éthylène, de siloxane, de phosphazène, d'alcoxysilanes, ou d'une céramique organiquement modifiée, ou un mélange d'au moins deux parmi les homopolymères et/ou copolymères susmentionnés et éventuellement d'au moins une céramique organiquement modifiée.

**16.** Mélange selon la revendication 15, **caractérisé en ce que** le composant b) est un homopolymère ou copolymère de difluorure de vinylidène, d'acrylonitrile, de (méth)acrylate de méthyle, de tétrahydrofurane, préférablement un homopolymère ou copolymère de difluorure de vinylidène.

**17.** Mélange selon l'une des revendications 13 à 16, **caractérisé en ce que** le polymère est au moins partiellement réticulé.

**18.** Mélange selon l'une des revendications 13 à 17, **caractérisé en ce qu'**il comprend en outre au moins un solvant.

**19.** Mélange selon la revendication 18, **caractérisé en ce que** les solvants présents sont des carbonates organiques, préférablement le carbonate d'éthylène, le carbonate de propylène, le carbonate de butylène, le carbonate de diméthyle, le carbonate de diéthyle, le carbonate d'éthyle et de méthyle ou le carbonate de méthyle et de propyle, des esters organiques, préférablement le formiate de méthyle, le formiate d'éthyle, l'acétate de méthyle, l'acétate d'éthyle, le propionate de méthyle, le propionate d'éthyle, le butyrate de méthyle, le butyrate d'éthyle, la $\gamma$-butyrolactone, des éthers organiques, préférablement l'éther diéthylique, le diméthoxyéthane, le diéthoxyéthane, des amides organiques, préférablement le diméthylformamide ou le diméthylacétamide, des solvants soufrés, préférablement le diméthylsulfoxyde, le diméthylsulfite, le diéthylsulfite ou la propane sultone, des solvants aprotiques, préférablement l'acétonitrile, l'acrylonitrile ou l'acétone, ou des dérivés au moins partiellement fluorés des solvants susmentionnés, ou des mélanges d'au moins deux parmi ces solvants et/ou de dérivés fluorés de ces solvants.

**20.** Procédé de préparation d'un mélange selon l'une des revendications 13 à 19, **caractérisé en ce qu'**au moins un sel de tétrakisfluoroalkylborate de formule générale (I) selon l'une des revendications 1 à 8, y compris les sels exclus ci-dessus, et au moins un polymère et éventuellement au moins un solvant, sont mélangés.

**21.** Procédé selon la revendication 20, **caractérisé en ce que** le mélange est effectué à une température élevée, préférablement à de 20 à 90°C, particulièrement préférablement à de 40 à 60°C.

**22.** Utilisation d'au moins un sel de tétrakisfluoroalkylborate de formule générale (I) selon l'une des revendications 1 à 8 ou d'au moins un mélange selon l'une des revendications 13 à 19 dans des électrolytes, des batteries primaires, des batteries rechargeables, des condensateurs, des super-condensateurs ou des cellules galvaniques, éventuellement aussi en combinaison avec d'autres sels conducteurs et/ou additives.

**23.** Electrolytes comprenant au moins un sel de tétrakisfluoroalkylborate de formule générale (I) selon l'une des revendications 1 à 8 ou au moins un mélange selon l'une des revendications 13 à 19.

**24.** Electrolytes selon la revendication 23, **caractérisés en ce que** la concentration en sel(s) de tétrakisfluoroalkylborate dans l'électrolyte va de 0,01 à 3 mol/l, préférablement de 0,01 à 2 mol/l, particulièrement préférablement de 0,1 à 1.5 mol/l.

**25.** Batteries primaires contenant au moins un sel de tétrakisfluoroalkylborate de formule générale (I) selon l'une des revendications 1 à 8 ou au moins un mélange selon l'une des revendications 13 à 19.

**26.** Batteries rechargeables contenant au moins un sel de tétrakisfluoroalkylborate de formule générale (I) selon l'une des revendications 1 à 8 ou au moins un mélange selon l'une des revendications 13 à 19.

**27.** Condensateurs contenant au moins un sel de tétrakisfluoroalkylborate de formule générale (I) selon l'une des

revendications 1 à 8 ou au moins un mélange selon l'une des revendications 13 à 19.

**28.** Super-condensateurs contenant au moins un sel de tétrakisfluoroalkylborate de formule générale (I) selon l'une des revendications 1 à 8 ou au moins un mélange selon l'une des revendications 13 à 19.

**29.** Cellules galvaniques contenant au moins un sel de tétrakisfluoroalkylborate de formule générale (I) selon l'une des revendications 1 à 8 ou au moins un mélange selon l'une des revendications 13 à 19.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4505997 A
- US 5273840 A
- EP 1174941 A
- DE 10042149
- DE 9941566
- DE 19953051
- DE 19932317
- DE 19951804
- DE 19959722
- DE 10027626
- DE 10008955
- DE 10016801
- DE 10026565
- DE 10027995
- DE 10016024
- DE 19922522
- DE 19946066
- DE 10014884
- DE 10025761
- DE 10025762


### In der Beschreibung aufgeführte Nicht-Patentliteratur


- **E. Bernhardt ; G. Henkel ; H. Willner ; Z. Anorg.** *Allg. Chem,* 2000, vol. 626, 560